# EUROPÄISCHE PATENTSCHRIFT

(11) **EP 4 344 681 B1**
(45) Veröffentlichungstag und Bekanntmachung des Hinweises auf die Patenterteilung: **21.10.2026**
(21) Anmeldenummer: 23193095.9
(22) Anmeldetag: 24.08.2023
(51) Int. Cl.: A61F 2/30, A61F 2/38, A61F 2/46, A61B 17/15

(54) **MODULARES INSTRUMENTENSYSTEM ZUR VERWENDUNG BEI EINER KNIEGELENKERSATZOPERATION**
MODULAR INSTRUMENTATION SYSTEM FOR USE IN KNEE JOINT REPLACEMENT SURGERY
SYSTÈME D'INSTRUMENT MODULAIRE DESTINÉ À ÊTRE UTILISÉ DANS UNE OPÉRATION DE REMPLACEMENT DE GENOU

(30) Priorität: 27.09.2022 DE 102022210214
(43) Veröffentlichungstag der Anmeldung: 03.04.2024
(73) Patentinhaber: Aesculap AG, 78532 Tuttlingen (DE)
(72) Erfinder: Firmbach, Franz-Peter, 78576 Emmingen-Liptingen (DE); Zouaghi, Housseyn, 52000 Chaumont (FR); Bork, Jana, 78315 Radolfzell (DE)
(74) Vertreter: Patentanwälte Ruff, Wilhelm, Beier, Dauster & Partner mbB

(56) Entgegenhaltungen:
- WO-A1-2020/037380
- CN-A- 110 051 456
- US-A1- 2021 068 965
- US-B2- 9 149 206

## Beschreibung

Die Erfindung betrifft ein modulares Instrumentensystem zur Verwendung bei einer Kniegelenkersatzoperation.

Bei einer Kniegelenkersatzoperation (englisch: total knee arthroplasty (TKA)) werden abgenutzte oder anderweitig durch Krankheit oder einen Unfall beeinträchtigte Gelenkflächen des Femurs und/oder der Tibia durch künstliche Gelenkflächen einer Kniegelenkprothese ersetzt. Solche Kniegelenkprothesen umfassen üblicherweise eine Femur-Komponente, eine Tibia-Komponente und eine Meniskus-Komponente. Die Femur-Komponente wird am distalen Ende des Femurs implantiert. Die Tibia-Komponente wird am proximalen Ende der Tibia implantiert. Die Meniskus-Komponente wird oftmals auch als Gleitfläche oder Knorpelersatz bezeichnet und zwischen der Femur- und der Tibia-Komponente angeordnet.

Vor der Implantation der prothetischen Komponenten werden der distale Femur und die proximale Tibia reseziert. Hierfür bringt der Chirurg unterschiedliche Resektionsschnitte an und trennt Knochen- und/oder Knorpelmaterial von dem jeweiligen Knochen ab. Durch die Resektion wird der jeweilige Knochen in seiner Form an die aufzunehmende prothetische Komponente angepasst.

Dabei wird in der TKA grundsätzlich zwischen zwei unterschiedlichen chirurgischen Herangehensweisen unterschieden, die auch als Measured Resection Technique (MRT) und Gap Balancing (GB) bekannt sind.

Bei der MRT wird der jeweilige Knochen um die Implantatdicke reseziert und anschließend eine Bandentspannung (englisch: ligament release) durchgeführt, bei welcher einzelne Bänder des Knies durchtrennt oder gedehnt werden. Hierdurch sollen insgesamt ausbalancierte Bandspannungen bei der Bewegung des Kniegelenks erreicht werden.

Bei dem Gap Balancing wird, im Gegensatz zu der MRT, der Resektionsschnitt des Femurs an die vorherrschende Bandspannung angepasst, was unter Umständen zu medial und lateral unterschiedlichen Resektionshöhen führt. Dabei wird zwischen der sogenannten Extension Gap Technique (EGT) und der Flexion Gap Technique (FGT) unterschieden. Bei der EGT wird der Flexionsspalt an den Extensionsspalt angepasst. Bei der FGT wird umgekehrt der Extensionsspalt an den Flexionsspalt angepasst. Unabhängig von der Herangehensweise werden stets der Extensions- und der Flexionsspalt gemessen.

Aus der US 9 149 206 B2 ist modulares Instrumentensystem in Form eines tibialen Probeimplantatsystems bekannt, das eine Lagerkomponente, eine Basiskomponente, eine Plattenkomponente und eine Shimkomponente umfasst. Die Shimkomponente kann zwischen einer Unterseite der Lagerkomponente und einer Oberseite der Basiskomponente angeordnet werden. Es sind mehrere unterschiedlich dicke Shimkomponenten vorhanden. Zur Anpassung einer Gesamtdicke des Probeimplantatsystems werden die unterschiedlich dicken Shimkomponenten wahlweise verwendet und zwischen der Unterseite der Lagerkomponente und einer Oberseite der Basiskomponente angeordnet.

Aufgabe der Erfindung ist es, ein modulares Instrumentensystem der eingangs genannten Art bereitzustellen, das Vorteile gegenüber dem Stand der Technik bietet und insbesondere eine vielseitige Verwendbarkeit auch bei unterschiedlichen Operationstechniken erlaubt.

Diese Aufgabe wird durch das Bereitstellen eines modularen Instrumentensystems mit den Merkmalen des Anspruchs 1 gelöst. Vorteilhafte Ausgestaltungen sind Gegenstand der Unteransprüche. Deren Wortlaut wird durch Bezugnahme zum Inhalt der Beschreibung gemacht.

Das erfindungsgemäße modulare Instrumentensystem weist wenigstens eine Tibia-Komponente, eine erste Femur-Komponente, eine zweite Femur-Komponente und ein Abstandselement auf. Die unterschiedlichen Komponenten des modularen Instrumentensystems können auf unterschiedliche Weise miteinander kombiniert verwendet werden. Dabei ist das modulare Instrumentensystem zum Ausbilden wenigstens einer ersten Instrumentenkonfiguration und einer zweiten Instrumentenkonfiguration eingerichtet. Die erste Instrumentenkonfiguration ist zur Verwendung bei wenigstens einer ersten Operationstechnik vorgesehen. Die zweite Instrumentenkonfiguration ist zur Verwendung bei wenigstens einer zweiten Operationstechnik vorgesehen. Durch die erfindungsgemäße Lösung kann das modulare Instrumentensystem besonders vielseitig verwendet werden. Dies bietet vielfältige Vorteile gegenüber aus dem Stand der Technik bekannten Spacer-Systemen, die üblicherweise lediglich für eine einzige Operationstechnik verwendbar sind. Die Tibia-Komponente weist eine Oberseite und eine, vorzugsweise parallel, gegenüberliegende Unterseite auf. Die Unterseite weist eine distal orientierte ebene Anlagefläche auf. Die Anlagefläche der Unterseite der Tibia-Komponente ist zur Anlage an einer resezierten proximalen Tibia eingerichtet. Die erste Femur-Komponente weist eine Oberseite und eine parallel gegenüberliegende Unterseite auf, wobei die Oberseite eine proximal orientierte ebene erste Anlagefläche aufweist. Die erste Anlagefläche ist um eine proximodistale erste Dicke von der Unterseite der ersten Femur-Komponente beabstandet und zur Anlage an einem resezierten Femur eingerichtet. Die erste Femur-Komponente kann an dem resezierten distalen Femur und an dem resezierten posterioren Femur angelegt werden. Hierdurch kann sowohl der Extensionsspalt als auch der Flexionsspalt gemessen werden. Die zweite Femur-Komponente weist eine Oberseite und eine parallel gegenüberliegende Unterseite auf, wobei die Oberseite eine proximal orientierte ebene zweite Anlagefläche aufweist. Die zweite Anlagefläche ist um eine proximodistale zweite Dicke von der Unterseite der zweiten Femur-Komponente beabstandet und zur Anlage an einem distalen Femur-Sägeblock und zur alternativen Anlage an einem posterioren Femur-Sägeblock eingerichtet. Je nach Instrumentenkonfiguration kommt entweder die erste Femur-Komponente oder die zweite Femur-Komponente zur Verwendung. Sollte der Femur noch nicht reseziert sein, ist es grundsätzlich denkbar, dass eine weitere Instrumentenkonfiguration lediglich die Tibiakomponente und das wenigstens eine Abstandselement umfasst und bei einer Operationstechnik zum Einsatz kommt, bei welcher nicht unbedingt gegen einen Sägeblock gemessen werden muss. Das Abstandselement weist eine Oberseite und eine um eine proximodistale Abstandsdicke parallel beabstandete Unterseite auf. Die Unterseite des Abstandselements weist einen distalen Verbindungsabschnitt auf. Der distale Verbindungsabschnitt des Abstandselements ist zur lösbaren Verbindung mit einem komplementären proximalen Verbindungsabschnitt der Oberseite der Tibia-Komponente eingerichtet. Die Oberseite des Abstandselements weist einen proximalen Verbindungsabschnitt auf. Dieser ist zur lösbaren Verbindung mit einem komplementären distalen Verbindungsabschnitt der Unterseite der ersten Femur-Komponente und zur alternativen lösbaren Verbindung mit einem komplementären distalen Verbindungsabschnitt der Unterseite der zweiten Femur-Komponente eingerichtet. Je nach erster oder zweiter Instrumentenkonfiguration ist der proximale Verbindungsabschnitt des Abstandselements entweder mit dem distalen Verbindungsabschnitt der ersten Femur-Komponente oder mit dem distalen Verbindungsabschnitt der zweiten Femur-Komponente lösbar verbunden. Die erste Femur-Komponente und die zweite Femur-Komponente unterscheiden sich in erster Linie hinsichtlich ihrer proximodistalen Dicke. Dabei bildet die erste Femur-Komponente die proximodistale Dicke eines Femur-Implantats oder auch Femur-Probeimplantats ab. Die erste Femur-Komponente, genauer: deren erste Anlagefläche, bildet eine definierte Anlage für den resezierten (distalen oder posterioren) Femur. Die in der zweiten Instrumentenkonfiguration zum Einsatz kommende zweite Femur-Komponente, genauer: deren zweite Anlagefläche, bietet eine definierte Anlage für den distalen Femur-Sägeblock oder alternativ für den posterioren Femur-Sägeblock. Gemeinsam mit dem posterioren Femur-Sägeblock bildet die zweite Femur-Komponente die anterioposteriore Dicke des Femur-Probeimplantats oder -implantats ab. Alternativ bildet die zweite Femur-Komponente gemeinsam mit dem distalen Femur-Sägeblock die proximodistale Dicke des besagten Femur-Probeimplantats und/oder Femur-Implantats ab. Die Verbindungsabschnitte der unterschiedlichen Komponenten des modularen Instrumentensystems sind vorzugsweise zur kraft- und/oder formschlüssigen Verbindung mit dem jeweils komplementären Verbindungsabschnitt eingerichtet. Vorzugsweise sind die Verbindungsabschnitte Steck-, Rast- und/oder Klemmabschnitte. Das Instrumentensystem kann zudem für ein sogenanntes Trialing verwendet werden.

Die in dieser Beschreibung verwendeten Lage- und Richtungsbezeichnungen beziehen sich auf den Körper eines Patienten, insbesondere dessen Tibia, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximodistale Achse alternativ als X-Achse bezeichnet werden. Die mediolaterale Achse kann als Y-Achse bezeichnet werden. Die anteroposteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Oberseite" in Bezug auf eine distal gerichtete Blickrichtung verwendet. Dementgegen werden Bezeichnungen wie "Unterseite" in Bezug auf eine proximal gerichtete Blickrichtung verwendet.

In Ausgestaltung der Erfindung ist die erste Dicke größer als die zweite Dicke. Mit anderen Worten ausgedrückt, ist die erste Femur-Komponente dicker als die zweite Femur-Komponente. Hierdurch kann eine anforderungsgerechte Referenzierung und/oder Anlage an dem resezierten distalen Femur (in Extension) oder resezierten posterioren Femur (in Flexion) einerseits und andererseits an dem distalen Femur-Sägeblock oder alternativ dem posterioren Femur-Sägeblock erreicht werden. Die erste Dicke der ersten Femur-Komponente beträgt vorzugsweise zwischen 7 mm und 11 mm, bevorzugt zwischen 8 mm und 10 mm, besonders bevorzugt 9 mm. Die zweite Dicke der zweiten Femur-Komponente beträgt vorzugsweise zwischen 4 mm und 6 mm, bevorzugt 5 mm. Bei einer bevorzugten Ausgestaltung beträgt die erste Dicke 9 mm und die zweite Dicke beträgt 5 mm.

In weiterer Ausgestaltung der Erfindung sind mehrere unterschiedlich dicke Abstandselemente mit unterschiedlichen Abstandsdicken vorhanden, wobei die mehreren unterschiedlich dicken Abstandselemente gegeneinander austauschbar einzeln und/oder miteinander kombiniert übereinandergestapelt verwendbar sind. Die unterschiedlich dicken Abstandselemente definieren unterschiedliche Dicken der zu implantierenden Gleitfläche. Durch ein Austauschen und/oder eine übereinandergestapelt kombinierte Verwendung der unterschiedlichen Abstandselemente kann die proximodistale Dicke der jeweiligen Instrumentenkonfiguration angepasst werden. Die Anpassung erfolgt mit dem Ziel einer ausgeglichenen Bänderspannung unter Extension und/oder Flexion. Bei einer Ausgestaltung umfassen die mehreren Abstandselemente zudem Abstandselemente mit identischer Abstandsdicke. Bei einer Ausgestaltung sind die unterschiedlichen Abstandselemente gegeneinander austauschbar einzeln verwendbar. In diesem Fall betragen die unterschiedlichen Abstandsdicken vorzugsweise 10 mm, 11 mm, 12 mm, 14 mm, 16 mm, 18 mm, 22 mm und 26 mm. Bei einer weiteren Ausgestaltung sind die unterschiedlichen Abstandselemente miteinander kombiniert übereinandergestapelt verwendbar. Bei dieser Ausgestaltung betragen die Abstandsdicken vorzugsweise 10 mm, 11 mm, 12 mm, 4 mm sowie 8 mm. Dabei können die Abstandselemente mit den Dicken 10 mm, 11 mm und 12 mm auch als "Base Plates" bezeichnet werden. Die Abstandselemente mit einer Abstandsdicke von beispielsweise 4 mm können auch als "Additional Height Plates" bezeichnet werden. Bei einer Ausgestaltung sind unterschiedliche "Additional Height Plates" mit unterschiedlichen Abstandsdicken vorhanden. Um eine erforderliche Gesamtdicke abzubilden, wird vorzugsweise eine der besagten Base Plates mit einer oder mehrerer Additional Height Plates übereinandergestapelt. Bei dieser Ausgestaltung weisen die übereinander stapelbaren Abstandselemente vorzugsweise zueinander komplementäre Verbindungsabschnitte auf. Mittels der komplementären Verbindungsabschnitte können die übereinandergestapelten Abstandselemente lösbar miteinander verbunden werden. Bevorzugt ist eine Steck-, Rast- und/oder Klemmverbindung.

In weiterer Ausgestaltung der Erfindung ist eine dritte Femur-Komponente vorhanden und weist eine Oberseite und eine um eine proximodistale dritte Dicke parallel beabstandete Unterseite auf, wobei die Oberseite einen proximalen Linearführungsabschnitt aufweist, welcher zur anteroposterior linearbeweglichen Führung an einem komplementären Linearführungsabschnitt eines Femur-Messblocks eingerichtet ist, wobei die Unterseite einen distalen Linearführungsabschnitt aufweist, welcher zur anteroposterior linearbeweglichen Führung an einem proximalen Linearführungsabschnitt der Oberseite des wenigstens einen Abstandselements eingerichtet ist, und wobei das modulare Instrumentensystem zum Ausbilden einer weiteren (dritten) Instrumentenkonfiguration eingerichtet ist, in welcher die dritte Femur-Komponente und das wenigstens eine Abstandselement mittels ihrer komplementären Linearführungsabschnitte lösbar relativ beweglich miteinander verbunden sind. Die dritte Instrumentenkonfiguration erlaubt die Verwendung bei wenigstens einer weiteren Operationstechnik. Dabei bildet die dritte Femur-Komponente die posteriore Dicke des Femur-Probeimplantats und/oder Femur-Implantats ab. Die dritte Femur-Komponente ist mittels der besagten Linearführungsabschnitte anteroposterior linearbeweglich geführt. Die Führung erfolgt einerseits an dem Femur-Messblock. Dieser ist nicht Bestandteil des modularen Instrumentensystems. Andererseits erfolgt die Führung an der Oberseite des wenigstens einen Abstandselements oder einem der mehreren unterschiedlichen Abstandselemente, sofern mehrere Abstandselemente vorhanden sind. Durch die Linearführung kann die dritte Femur-Komponente in der Verwendung anterior und posterior relativ zu dem Femur-Messblock und/oder dem Abstandselement verschoben werden. Dies ermöglicht eine besonders vorteilhafte Ermittlung des Gelenkspalts. Zudem ist die dritte Femur-Komponente mittels ihrer Linearführungsabschnitte verliersicher an den weiteren Komponenten der dritten Instrumentenkonfiguration gehalten. Hierdurch ergeben sich ergonomische Vorteile ("Free Hand Working"). Die dritte Dicke der dritten Femur-Komponente beträgt vorzugsweise zwischen 7 mm und 11 mm, bevorzugt zwischen 8 mm und 10 mm, besonders bevorzugt 9 mm.

In weiterer Ausgestaltung der Erfindung weist der proximale Linearführungsabschnitt der dritten Femur-Komponente wenigstens eine in die Oberseite distal eingesenkte und anteroposterior längserstreckte sowie mediolateral hinterschnittene Führungsnut auf. Die wenigstens eine Führungsnut ist zum gleitbeweglichen Zusammenwirken mit dem komplementären Linearführungsabschnitt des Femur-Messblocks eingerichtet. Durch den mediolateralen Hinterschnitt der Führungsnut ist der komplementäre Linearführungsabschnitt des Femur-Messblocks proximodistal formschlüssig in der Führungsnut gehalten.

In weiterer Ausgestaltung der Erfindung weist der distale Linearführungsabschnitt der dritten Femur-Komponente wenigstens einen von der Unterseite distal aufragenden Führungsstift auf, und der proximale Linearführungsabschnitt des wenigstens einen Abstandselements weist wenigstens eine in die Oberseite distal eingesenkte anteroposterior längserstreckte sowie mediolateral hinterschnittene Führungsnut auf. Der Führungsstift und die Führungsnut wirken anteroposterior gleitbeweglich und ansonsten formschlüssig zusammen. Zum Formschluss in proximodistaler Richtung ist die Führungsnut mediolateral hinterschnitten. Der Führungsstift ist vorzugsweise komplementär zu dem Hinterschnitt gestaltet. Beispielsweise weist der Führungsstift an seinem distalen Ende einen Pilzkopf, eine radiale Erweiterung oder dergleichen auf. Vorzugsweise weist die dritte Femur-Komponente zwei mediolateral voneinander beabstandete Führungsstifte auf. In diesem Fall weist das wenigstens eine Abstandselement vorzugsweise zwei mediolateral voneinander beabstandete Führungsnuten auf.

In weiterer Ausgestaltung der Erfindung ist wenigstens ein Augmentatselement vorhanden und weist eine proximale Augmentatsfläche zur Anlage an dem resezierten distalen Femur und einen distalen Verbindungsabschnitt auf, welcher zur lösbaren Verbindung mit einem proximalen Verbindungsabschnitt der ersten Femur-Komponente eingerichtet ist, wobei das modulare Instrumentensystem zum Ausbilden einer weiteren (vierten) Instrumentenkonfiguration eingerichtet ist, in welcher das Augmentatselement an der Anlagefläche der ersten Femur-Komponente angebracht ist. Mittels des wenigstens einen Augmentatselements kann ein etwaiger Defekt an dem resezierten Femur maßlich ausgeglichen werden. Dies ist insbesondere bei einem Revisionseingriff vorteilhaft. Die proximale Augmentatsfläche ist anteroposterior sowie mediolateral kleiner als die erste Anlagefläche der ersten Femur-Komponente. Mit anderen Worten ausgedrückt, wird die erste Anlagefläche lediglich lokal augmentiert. Der distale Verbindungsabschnitt des Augmentatselements und der komplementäre proximale Verbindungsabschnitt der ersten Femur-Komponente erlauben eine form- und/oder kraftschlüssige lösbare Verbindung. Bevorzugt ist eine Steck-, Rast- und/oder Klemmverbindung.

In weiterer Ausgestaltung der Erfindung weist die erste Femur-Komponente mehrere proximale Verbindungsabschnitte auf, die jeweils in die Oberseite der ersten Femur-Komponente eingesenkt und mediolateral und/oder anteroposterior voneinander beabstandet positioniert sind. Die mehreren proximalen Verbindungsabschnitte sind jeweils zur lösbaren Verbindung mit dem distalen Verbindungsabschnitt des wenigstens einen Augmentatselements eingerichtet. Hierdurch kann das Augmentatselement wahlweise in unterschiedlichen Positionen an der ersten Anlagefläche lösbar befestigt werden. Sofern mehrere Augmentatselemente vorhanden sind, können diese kombiniert an jeweils einem der proximalen Verbindungsabschnitte der ersten Femur-Komponente angebracht werden. Zudem ist es denkbar, dass mehrere Augmentatselemente übereinandergestapelt und miteinander verbunden an der ersten Femur-Komponente angebracht werden.

In weiterer Ausgestaltung der Erfindung sind mehrere unterschiedlich geformte und/oder unterschiedlich große Augmentatselemente vorhanden und gegeneinander austauschbar verwendbar. Die unterschiedlichen Augmentatselemente weisen bei einer Ausgestaltung eine unterschiedlich geformte Außenkontur auf. Bei einer weiteren Ausgestaltung sind die Augmentatselemente alternativ oder zusätzlich unterschiedlich groß, insbesondere im Hinblick auf ihre anteroposteriore und/oder mediolaterale und/oder proximodistale Erstreckung.

In weiterer Ausgestaltung der Erfindung ist ein Handgriff vorhanden und weist wenigstens eine manuell betätigbare Kopplungseinrichtung auf, welche zur lösbaren Kopplung mit einem Kopplungsabschnitt des wenigstens einen Abstandselements eingerichtet ist. Mittels des Handgriffs kann die jeweilige Instrumentenkonfiguration ergonomisch vorteilhaft in den Gelenkspalt eingeführt und aus diesem entnommen werden. Der Handgriff bietet insoweit in erster Linie ergonomische Vorteile. Zur Kopplung mit der jeweiligen Instrumentenkonfiguration weist der Handgriff wenigstens eine manuell betätigbare Kopplungseinrichtung auf. Die Kopplungseinrichtung ist zur lösbaren Kopplung mit dem komplementären Kopplungsabschnitt des wenigstens einen Abstandselements eingerichtet. Sofern das modulare Instrumentensystem mehrere unterschiedliche Abstandselemente aufweist, weist vorzugsweise jedes der Abstandselemente einen solchen Kopplungsabschnitt auf. Die Kopplung ist form- und/oder kraftschlüssig. Bei unterschiedlichen Ausgestaltungen bilden die Kopplungseinrichtung und der komplementäre Kopplungsabschnitt eine lösbare Steck-, Rast- und/oder Klemmverbindung aus. Vorzugsweise ist die Kopplungseinrichtung einends des Handgriffs angeordnet. Vorzugsweise ist die Kopplungseinrichtung manuell zwischen einem Kopplungszustand und einem Freigabezustand überführbar. In dem Kopplungszustand ist das wenigstens eine Abstandselement mittels der Kopplungseinrichtung lösbar mit dem Handgriff gekoppelt. In dem Freigabezustand ist die Kopplung freigegeben.

In weiterer Ausgestaltung der Erfindung ist der Handgriff zwischen einem ersten Ende und einem zweiten Ende längserstreckt und weist an beiden Enden jeweils eine Kopplungseinrichtung auf. Dies ist eine besonders bevorzugte Ausgestaltung der Erfindung. Durch die beiden Kopplungseinrichtungen kann der Handgriff beidseits mit jeweils einer Instrumentenkonfiguration gekoppelt werden. Die Instrumentenkonfigurationen können unterschiedliche Gesamtdicken aufweisen. Die auf diese Weise an den Handgriff gekoppelten Instrumentenkonfigurationen können zur Bestimmung des Gelenkspalts einfach und für den Chirurgen ergonomisch wechselweise in den Gelenkspalt eingebracht und aus diesem entnommen werden.

In weiterer Ausgestaltung der Erfindung ist wenigstens ein Tibia-Probeplateau vorhanden und weist eine Oberseite und eine distal gegenüberliegende Unterseite auf, wobei die Unterseite eine distal orientierte Befestigungsfläche zur Befestigung an der resezierten proximalen Tibia aufweist, wobei die Oberseite einen proximalen Verbindungsabschnitt aufweist, welcher zur lösbaren Verbindung mit dem distalen Verbindungsabschnitt des wenigstens einen Abstandselements eingerichtet ist, und wobei das modulare Instrumentensystem zum Ausbilden einer weiteren (fünften) Instrumentenkonfiguration eingerichtet ist, in welcher das wenigstens eine Abstandselement und das Tibia-Probeplateau miteinander lösbar verbunden sind. Vereinfacht ausgedrückt, können die Tibia-Komponente und das Tibia-Probeplateau gegeneinander ausgetauscht werden. Im Unterschied zu der Tibia-Komponente ist das Tibia-Probeplateau zur Befestigung an der proximalen Tibia eingerichtet. Die Tibia-Komponente ist lediglich zur Anlage an der proximalen Tibia eingerichtet und insoweit in der Verwendung anteroposterior sowie mediolateral relativbeweglich. Der proximale Verbindungsabschnitt an der Oberseite des Tibia-Probeplateaus ist vorzugsweise identisch zu dem proximalen Verbindungsabschnitt an der Oberseite der Tibia-Komponente. Die weitere (fünfte) Instrumentenkonfiguration ist zur Verwendung gemäß wenigstens einer weiteren Operationstechnik vorgesehen.

In weiterer Ausgestaltung der Erfindung sind mehrere unterschiedlich große Tibia-Probeplateaus vorhanden und gegeneinander austauschbar verwendbar. Die Tibia-Probeplateaus unterscheiden sich vorzugsweise hinsichtlich ihrer mediolateralen und/oder anteroposterioren Abmessungen. Hierzu im Unterschied liegt die Tibia-Komponente vorzugsweise lediglich in einer einzigen Größe vor. Durch die mehreren unterschiedlich großen Tibia-Probeplateaus kann das modulare Instrumentensystem an unterschiedlich großen Tibia-Knochen verwendet werden.

In weiterer Ausgestaltung der Erfindung ist wenigstens ein Tibia-Plateau vorhanden und weist eine Oberseite und einen distalen Verankerungszapfen zur Verankerung an der resezierten proximalen Tibia auf, wobei die Oberseite einen proximalen Verbindungsabschnitt aufweist, welcher zur lösbaren Verbindung mit dem distalen Verbindungsabschnitt des wenigstens einen Abstandselements eingerichtet ist, und wobei das modulare Instrumentensystem zum Ausbilden einer weiteren (sechsten) Instrumentenkonfiguration eingerichtet ist, in welcher das wenigstens eine Abstandselement und das Tibia-Plateau miteinander lösbar verbunden sind. Durch das wenigstens eine Tibia-Plateau kann das modulare Instrumentensystem in besonders vielseitiger Weise verwendet werden. Vereinfacht ausgedrückt, ist das Tibia-Plateau gegen die Tibia-Komponente und/oder das Tibia-Probeplateau austauschbar und umgekehrt. Die weitere (sechste) Instrumentenkonfiguration ist zur Verwendung gemäß wenigstens einer weiteren Operationstechnik und/oder zur Verwendung bei einer oder mehrerer der bereits erwähnten Operationstechniken vorgesehen.

In weiterer Ausgestaltung der Erfindung sind mehrere unterschiedlich große Tibia-Plateaus vorhanden und gegeneinander austauschbar verwendbar. Die unterschiedlich großen Tibia-Plateaus weisen unterschiedliche anteroposteriore und/oder mediolaterale Abmessungen auf.

Durch die mehreren unterschiedlich großen Tibia-Plateaus kann das modulare Instrumentensystem an unterschiedlich großen Tibia-Knochen verwendet werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform eines erfindungsgemäßen modularen Instrumentensystems,
- Fig. 2: in schematischer Perspektivdarstellung eine Tibia-Komponente des Instrumentensystems nach Fig. 1,
- Fig. 3: in schematischer Perspektivdarstellung ein Tibia-Probeplateau des Instrumentensystems nach Fig. 1,
- Fig. 4: in schematischer Perspektivdarstellung ein Tibia-Plateau des Instrumentensystems nach Fig. 1,
- Fig. 5: eine schematische Draufsicht eines Abstandselements des Instrumentensystems nach Fig. 1,
- Fig. 6: das Abstandselement nach Fig. 5 in einer schematischen Unteransicht,
- Fig. 7: in schematischer Perspektivdarstellung eine Instrumentenkonfiguration des Instrumentensystems nach Fig. 1, in welcher das Abstandselement lösbar mit der Tibia-Komponente verbunden ist,
- Fig. 8: in schematischer Perspektivdarstellung eine weitere Instrumentenkonfiguration des Instrumentensystems nach Fig. 1, in welcher das Abstandselement lösbar mit dem Tibia-Probeplateau verbunden ist,
- Fig. 9: in schematischer Perspektivdarstellung eine weitere Instrumentenkonfiguration des Instrumentensystems nach Fig. 1, in welcher das Abstandselement lösbar mit dem Tibia-Plateau verbunden ist,
- Fig. 10: in schematischer Perspektivdarstellung eine weitere Instrumentenkonfiguration des Instrumentensystems nach Fig. 1, in welcher ein Handgriff des Instrumentensystems beidseits mit jeweils einem Abstandselement des Instrumentensystems lösbar gekoppelt ist,
- Fig. 11 bis 16: unterschiedliche schematische Perspektivdarstellungen zur Verdeutlichung einer Variante des Instrumentensystems nach Fig. 1,
- Fig. 17: in schematischer Perspektivdarstellung eine erste Femur-Komponente des Instrumentensystems nach Fig. 1,
- Fig. 18: eine schematische Unteransicht der ersten Femur-Komponente nach Fig. 17,
- Fig. 19: eine schematische Draufsicht der ersten Femur-Komponente nach den Fig. 17
- und: 18,
- Fig. 20: in schematischer Perspektivdarstellung eine weitere Instrumentenkonfiguration des Instrumentensystems nach Fig. 1, in welcher die erste Femur-Komponente lösbar mit dem Abstandselement und der Tibia-Komponente verbunden ist,
- Fig. 21: in schematischer Perspektivdarstellung ein Augmentatselement des Instrumentensystems nach Fig. 1,
- Fig. 22: in schematischer Perspektivdarstellung eine weitere Instrumentenkonfiguration des Instrumentensystems nach Fig. 1, in welcher das Augmentatselement lösbar an einer Oberseite der ersten Femur-Komponente angebracht ist,
- Fig. 23: in schematischer Perspektivdarstellung ein weiteres Augmentatselement des Instrumentensystems nach Fig. 1,
- Fig. 24: in schematischer Perspektivdarstellung eine weitere Instrumentenkonfiguration des Instrumentensystems nach Fig. 1, in welcher das weitere Augmentatselement lösbar an der Oberseite der ersten Femur-Komponente angebracht ist,
- Fig. 25: in schematischer Perspektivdarstellung eine zweite Femur-Komponente des Instrumentensystems nach Fig. 1,
- Fig. 26: eine weitere schematische Perspektivdarstellung der zweiten Femur-Komponente nach Fig. 25,
- Fig. 27: in schematischer Perspektivdarstellung eine weitere Instrumentenkonfiguration des Instrumentensystems nach Fig. 1, in welcher die zweite Femur-Komponente lösbar mit dem Abstandselement und der Tibia-Komponente verbunden ist,
- Fig. 28: in schematischer Perspektivdarstellung eine dritte Femur-Komponente des Instrumentensystems nach Fig. 1,
- Fig. 29: eine weitere schematische Perspektivdarstellung der dritten Femur-Komponente nach Fig. 28,
- Fig. 30: in schematischer Perspektivdarstellung die dritte Femur-Komponente zusammen mit dem Abstandselement und der Tibia-Komponente,
- Fig. 31: in schematischer Perspektivdarstellung eine weitere Instrumentenkonfiguration des Instrumentensystems nach Fig. 1, in welcher die dritte Femur-Komponente anteroposterior linearbeweglich geführt an dem Abstandselement festgelegt und mittelbar mit der Tibia-Komponente verbunden ist, und
- Fig. 32: in schematischer Perspektivdarstellung die Instrumentenkonfiguration nach Fig. 31 zusammen mit einem Femur-Messblock.

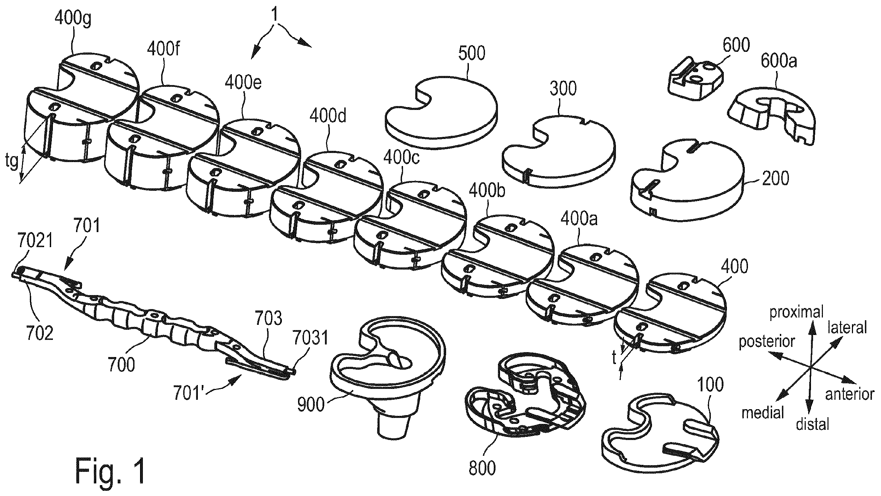

Gemäß Fig. 1 ist ein modulares Instrumentensystem 1 zur Verwendung bei einer Kniegelenkersatzoperation vorgesehen. Das modulare Instrumentensystem 1 kann auch als modulares Spacer-System bezeichnet werden und dient zur Bestimmung und/oder Ausbalancierung eines Gelenkspalts zwischen der Tibia und dem Femur bei einer totalkondylären-Kniearthroplastie (TKA). Mittels des Spacer-Systems kann, beispielsweise im Rahmen eines Gap Balancing (GP), unter anderem eine erforderliche proximodistale Dicke einer Meniskuskomponente (Gleitfläche) einer zu implantierenden Kniegelenkprothese ermittelt sowie die Stabilität des Kniegelenks in Extension oder Flexion geprüft werden. Dabei erlaubt das modulare Spacer-System 1 die Anwendung unterschiedlicher Operationstechniken. Hierfür werden unterschiedliche noch näher zu erläuternde Komponenten des modularen Spacer-Systems 1 unter Ausbildung unterschiedlicher Instrumentenkonfigurationen lösbar miteinander verbunden.

Bei der gezeigten Ausführungsform weist das modulare Instrumentensystem 1 eine Tibia-Komponente 100, eine erste Femur-Komponente 200, eine zweite Femur-Komponente 300, wenigstens ein Abstandselement 400, eine dritte Femur-Komponente 500, wenigstens ein Augmentatselement 600, einen Handgriff 700, ein Tibia-Probeplateau 800 sowie ein Tibia-Plateau 900 auf.

Nicht sämtliche der vorgenannten Komponenten des modularen Instrumentensystems 1 sind im Hinblick auf die vorliegende Erfindung als wesentlich zu erachten. Dementsprechend sind bei unterschiedlichen - in den Figuren nicht gezeigten - Ausführungsformen nicht sämtliche der besagten Komponenten vorhanden. Bei einer in den Figuren nicht gezeigten Ausführungsform weist das modulare Instrumentensystem keine dritte Femur-Komponente und/oder kein Augmentatselement und/oder keinen Handgriff und/oder kein Tibia-Probeplateau und/oder kein Tibia-Plateau auf. Die anhand Fig. 1 gezeigte Zusammenstellung der Komponenten ist insoweit als rein exemplarisch zu erachten.

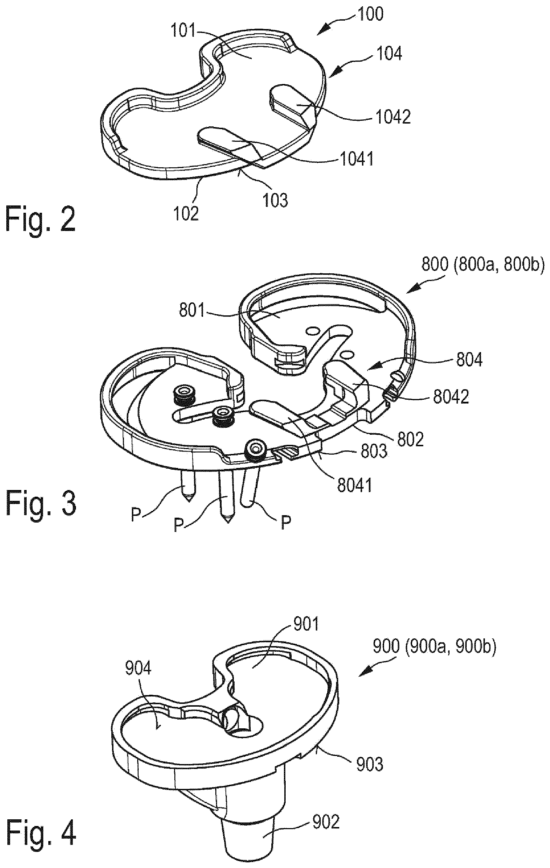

Die Tibia-Komponente 100 (Fig. 2) weist eine Oberseite 101 und eine distal gegenüberliegende Unterseite 102 auf. Die Unterseite 102 weist eine distal orientierte Anlagefläche 103 auf. Die Anlagefläche 103 ist eben und zur Anlage an einer resezierten proximalen Tibia eingerichtet. In der Verwendung der Tibia-Komponente 100 ist die Anlagefläche 103 anteroposterior und/oder mediolateral relativbeweglich zu der resezierten proximalen Tibia. Die Oberseite 101 der Tibia-Komponente 100 weist einen proximalen Verbindungsabschnitt 104 auf. Der proximale Verbindungsabschnitt 104 ist zur lösbaren Verbindung mit einem komplementären distalen Verbindungsabschnitt des wenigstens einen Abstandselements 400 eingerichtet. Bei der gezeigten Ausführungsform weist der proximale Verbindungsabschnitt 104 Teilabschnitte 1041, 1042 auf. Je nachdem, ob eine Verwendung an einem linken oder rechten Knie stattfindet, können die Teilabschnitte 1041, 1042 auch als medialer Verbindungsteilabschnitt 1041 und lateraler Verbindungsteilabschnitt 1042 bezeichnet werden oder umgekehrt. Die Teilverbindungsabschnitte 1041, 1042 sind vorliegend anterior an der Oberseite 101 angeordnet. Die spezifische Gestaltung des Verbindungsabschnitts 104 und/oder der Verbindungsteilabschnitte 1041, 1042 ist im Hinblick auf die vorliegende Erfindung nicht von wesentlicher Bedeutung. Entscheidend ist, dass der proximale Verbindungsabschnitt 104 eine lösbare Verbindung mit dem komplementären distalen Verbindungsabschnitt des wenigstens einen Abstandselements 400 gestattet.

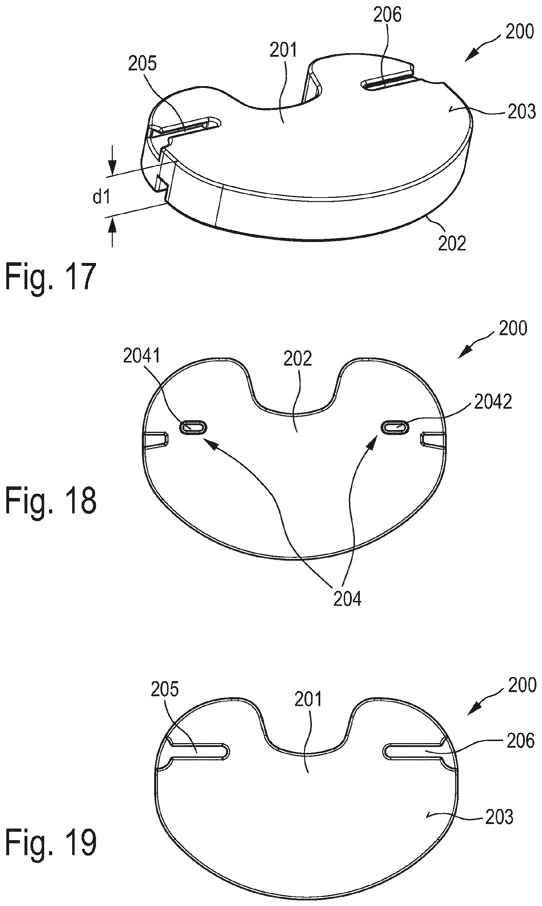

Die erste Femur-Komponente 200 (siehe Fig. 17, 18, 19) weist eine Oberseite 201 und eine parallel gegenüberliegende Unterseite 202 auf. Die Oberseite 201 weist eine proximal orientierte ebene erste Anlagefläche 203 auf. Die erste Anlagefläche 203 ist um eine proximodistale erste Dicke d1 von der Unterseite 202 beabstandet. Die erste Anlagefläche 203 ist zur Anlage an einem resezierten distalen Femur und/oder einem resezierten posterioren Femur eingerichtet. Die erste Dicke d1 repräsentiert hierbei die proximodistale Dicke des zu implantierenden Femur-Probeimplantats und/oder Femur-Implantats. Die Unterseite 202 der ersten Femur-Komponente 200 weist einen distalen Verbindungsabschnitt 204 auf. Der distale Verbindungsabschnitt 204 ist auf noch näher beschriebene Weise zur lösbaren Verbindung mit einem komplementären proximalen Verbindungsabschnitt des wenigstens einen Abstandselements 400 eingerichtet. Bei der gezeigten Ausführungsform weist der distale Verbindungsabschnitt 204 zwei mediolateral voneinander beabstandete Verbindungsteilabschnitte 2041, 2042 auf. Die spezifische Gestaltung des distalen Verbindungsabschnitts 204 ist im Hinblick auf die vorliegende Erfindung nicht von wesentlicher Bedeutung. Entscheidend ist, dass der distale Verbindungsabschnitt 204 eine lösbare Verbindung mit dem komplementären proximalen Verbindungsabschnitt des wenigstens einen Abstandselements 400 erlaubt.

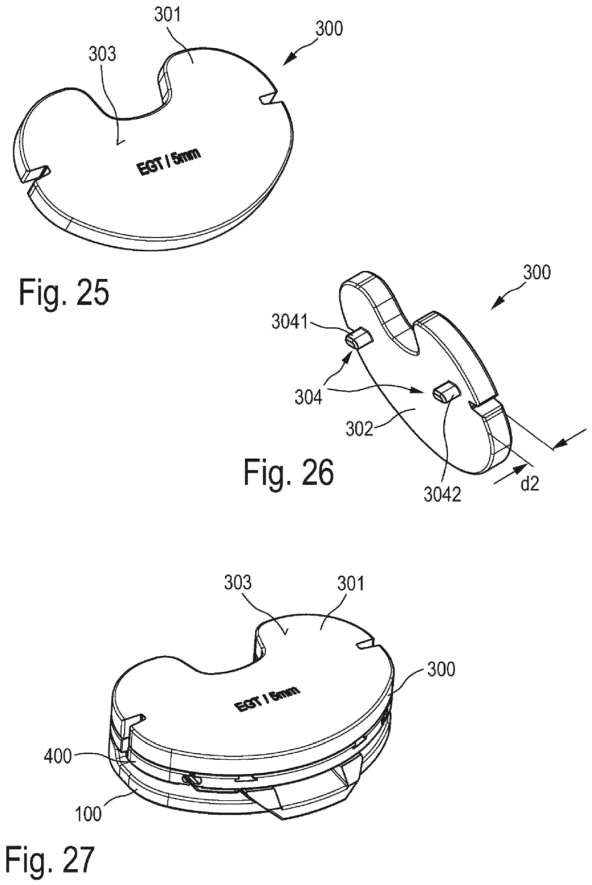

Die zweite Femur-Komponente 300 (siehe Fig. 25, 26, 27) weist eine Oberseite 301 und eine parallel gegenüberliegende Unterseite 302 auf. Die Oberseite 301 weist eine proximal orientierte ebene zweite Anlagefläche 303 auf. Die zweite Anlagefläche 303 ist um eine proximodistale zweite Dicke d2 von der Unterseite 302 beabstandet. Dabei ist die zweite Anlagefläche zur Anlage an einem distalen Femur-Sägeblock und zur alternativen Anlage an einem posterioren Femur-Sägeblock eingerichtet. Sowohl der distale Femur-Sägeblock als auch der posteriore Femur-Sägeblock sind nicht Bestandteil des modularen Instrumentensystems 1. Der Aufbau und die Funktion solcher Sägeblöcke sind dem zuständigen Fachmann prinzipiell bekannt. Weitere dahingehende Erläuterungen sind daher entbehrlich. Die Unterseite 302 der zweiten Femur-Komponente 300 weist einen distalen Verbindungsabschnitt 304 auf. Der distale Verbindungsabschnitt 304 ist zur lösbaren Verbindung mit dem bereits erwähnten komplementären proximalen Verbindungsabschnitt des wenigstens einen Abstandselements 400 eingerichtet. Bei der gezeigten Ausführungsform weist der distale Verbindungsabschnitt 304 zwei mediolateral voneinander beabstandete Verbindungsteilabschnitte 3041, 3042 auf. Hinsichtlich der weiteren Gestaltung des distalen Verbindungsabschnitts 304 gilt mutatis mutandis das zu dem distalen Verbindungsabschnitt 204 der ersten Femur-Komponente 200 Gesagte. Die erste Femur-Komponente 200 und die zweite Femur-Komponente 300 können wahlweise mit dem wenigstens einen Abstandselement 400 verbunden werden. Die distalen Verbindungsabschnitte 204, 304 sind dementsprechend identisch gestaltet.

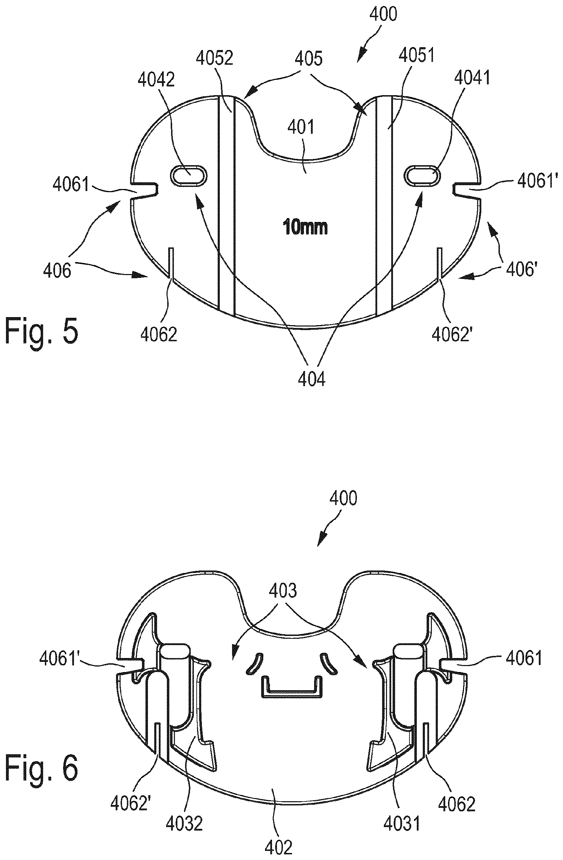

Das wenigstens eine Abstandselement (siehe Fig. 5, 6) weist eine Oberseite 401 und eine parallel gegenüberliegende Unterseite 402 auf. Die Oberseite 401 ist proximal orientiert. Die Unterseite 402 ist distal orientiert. Die Oberseite 401 und die Unterseite 402 sind um eine proximodistale Abstandsdicke t (siehe Fig. 1) voneinander beabstandet. Mit anderen Worten ausgedrückt, weist das Abstandselement 400 eine proximodistale Dicke t auf. Die Oberseite 401 weist einen proximalen Verbindungsabschnitt 404 auf. Der proximale Verbindungsabschnitt 404 ist zur lösbaren Verbindung mit dem distalen Verbindungsabschnitt 204 der ersten Femur-Komponente 200 (siehe Fig. 18) oder wahlweise zur lösbaren Verbindung mit dem distalen Verbindungsabschnitt 304 der zweiten Femur-Komponente 300 (siehe Fig. 26) eingerichtet. Die besagten proximalen und distalen Verbindungsabschnitte sind in ihrer Form und/oder Abmessung dementsprechend aufeinander abgestimmt. Der proximale Verbindungsabschnitt 404 der Oberseite 401 des Abstandselements 400 weist dementsprechend zwei mediolateral voneinander beabstandete Verbindungsteilabschnitte 4041, 4042 auf.

Bei der gezeigten Ausführungsform ist der proximale Verbindungsabschnitt 404 distal in die Oberseite 401 des Abstandselements 400 eingesenkt. Die distalen Verbindungsabschnitte 204, 304 ragen hierzu komplementär distal von der jeweiligen Unterseite 202, 302 ab. Im Speziellen können die Verbindungsteilabschnitte 4041, 4042 jeweils auch als Steckaufnahme bezeichnet werden. Dementsprechend können die komplementären Verbindungsteilabschnitte 2041, 2042, 3041, 3042 jeweils auch als Steckelement bezeichnet werden. Bei der gezeigten Ausführungsform ist die lösbare Verbindung zwischen dem Abstandselement 400 und der jeweiligen Femur-Komponente 200, 300 dementsprechend eine lösbare Steckverbindung. In einem miteinander verbundenen Zustand sind die besagten Komponenten anteroposterior und/oder mediolateral formschlüssig relativ zueinander festgelegt. Zudem sind die besagten Komponenten proximodistal lösbar aneinander gehalten.

Die Unterseite 402 des Abstandselements 400 weist einen distalen Verbindungsabschnitt 403 auf. Der distale Verbindungsabschnitt 403 ist zur lösbaren Verbindung mit dem proximalen Verbindungsabschnitt 104 der Tibia-Komponente 100 (siehe Fig. 2) eingerichtet. Die besagten Verbindungsabschnitte sind zueinander komplementär. Dementsprechend weist der distale Verbindungsabschnitt 403 zwei mediolateral voneinander beabstandet angeordnete Verbindungsteilabschnitte 4031, 4032 auf. Die distalen Verbindungsteilabschnitte 4031, 4032 können zur lösbaren Verbindung mit der Tibia-Komponente 100 mit dem jeweiligen proximalen Verbindungsteilabschnitt 1041, 1042 verrastet werden.

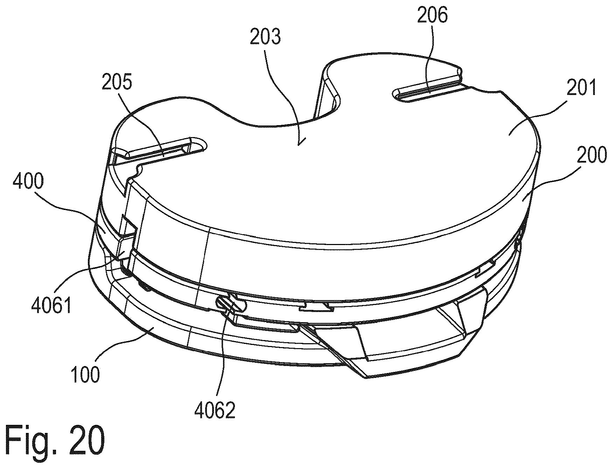

Fig. 20 zeigt eine unter Verwendung des modularen Instrumentensystems 1 ausgebildete Instrumentenkonfiguration. Die erste Instrumentenkonfiguration weist die Tibia-Komponente 100, das wenigstens eine Abstandselement 400 und die erste Femur-Komponente 200 auf.

Fig. 27 zeigt eine weitere Instrumentenkonfiguration. Die weitere Instrumentenkonfiguration weist die Tibia-Komponente 100, das wenigstens eine Abstandselement 400 und die zweite Femur-Komponente 300 auf.

Die beiden Instrumentenkonfigurationen nach Fig. 20 und Fig. 27 können auch als erste Instrumentenkonfiguration und zweite Instrumentenkonfiguration bezeichnet werden. Jede der Instrumentenkonfigurationen kann einem oder mehreren unterschiedlichen Workflows und/oder einer oder mehreren Operationstechniken zugeordnet sein. Im Speziellen ist die zweite Instrumentenkonfiguration vorliegend einem Gap Balancing, genauer: einem EGT, zugeordnet. Die erste Instrumentenkonfiguration kann vorliegend bei unterschiedlichen Operationstechniken verwendet werden. In der ersten Instrumentenkonfiguration ist die erste Femur-Komponente 200 lösbar mit dem wenigstens einen Abstandselement 400 verbunden. Dieses ist wiederum lösbar mit der Tibia-Komponente 100 verbunden. In der zweiten Instrumentenkonfiguration ist die zweite Femur-Komponente 300 lösbar mit dem wenigstens einen Abstandselement 400 verbunden. Dieses ist wiederum lösbar mit der Tibia-Komponente 100 verbunden.

Bei der gezeigten Ausführungsform beträgt die erste Dicke d1 der ersten Femur-Komponente 200 9 mm. Die zweite Dicke d2 der zweiten Femur-Komponente 300 beträgt 5 mm. Dementsprechend ergeben sich in den unterschiedlichen Instrumentenkonfigurationen nach Fig. 20 und Fig. 27 unterschiedliche Gesamtdicken.

Bei der gezeigten Ausführungsform weist das modulare Instrumentensystem 1 mehrere unterschiedlich dicke Abstandselemente 400, 400a bis 400g auf (siehe Fig. 1). Abgesehen von ihrer jeweiligen proximodistalen Dicke sind die Abstandselemente 400, 400a bis 400g vorliegend identisch hinsichtlich ihrer Gestaltung und Funktion, so dass gegenständliche und/oder funktionelle Merkmale der Abstandselemente 400, 400a bis 400g nachfolgend nicht gesondert für jedes einzelne der Abstandselemente erläutert werden. Die unterschiedlichen Abstandselemente 400, 400a bis 400g sind gegeneinander austauschbar einzeln verwendbar. Beispielsweise kann eines der Abstandselemente 400a bis 400g anstelle des Abstandselements 400 in der ersten Instrumentenkonfiguration (Fig. 20) und/oder der zweiten Instrumentenkonfiguration (Fig. 27) verwendet werden. Das jeweilige Abstandselement 400, 400a bis 400g definiert die Gesamtdicke der Instrumentenkonfiguration. Mit anderen Worten ausgedrückt, kann durch das Austauschen der Abstandselemente 400, 400a bis 400g die Dicke der jeweiligen Instrumentenkonfiguration zum Zwecke der Ermittlung und/oder Ausbalancierung des Gelenkspalts angepasst werden.

Bei der gezeigten Ausführungsform sind die proximodistalen Dicken der Abstandselemente 400, 400a bis 400g wie folgt gestaffelt: 10 mm, 11 mm, 12 mm, 14 mm, 16 mm, 18 mmm, 22 mm und 26 mm. Dementsprechend beträgt die Abstandsdicke t 10 mm. Die Abstandsdicke tg des Abstandselements 400g beträgt 26 mm.

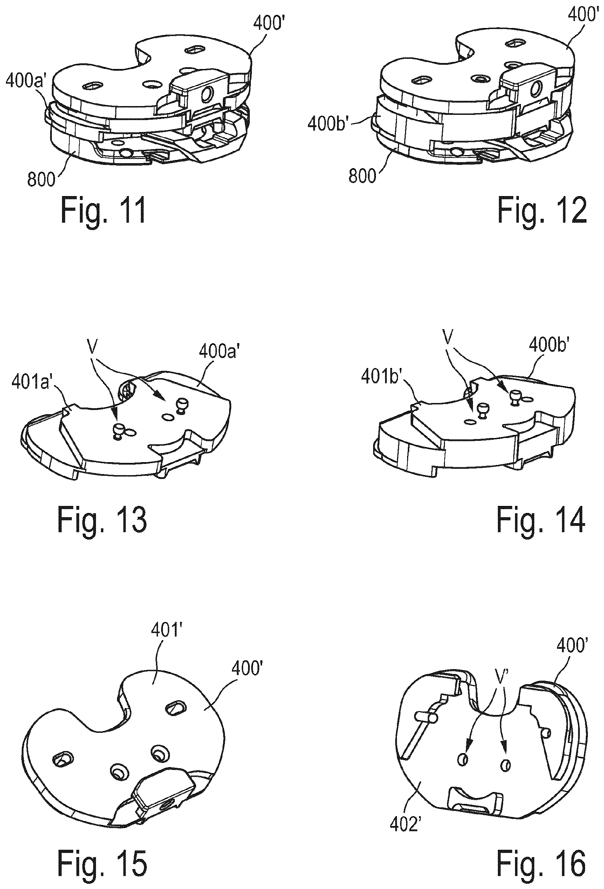

Anstelle einer einzelnen und gegeneinander austauschbaren Verwendung der Abstandselemente ist auch eine kombinierte, übereinandergestapelte Verwendung denkbar. Eine solche alternative Gestaltung ist anhand der Fig. 11 bis 16 verdeutlicht. Dort sind allein zur Verdeutlichung drei unterschiedliche Abstandselemente 400a', 400b' und 400' gezeigt. Die beiden Abstandselemente 400a' und 400b' können auch als erste Zusatzplatte und zweite Zusatzplatte bezeichnet werden. Die beiden Zusatzplatten unterscheiden sich insbesondere im Hinblick auf ihre proximodistale Dicke und sind gegeneinander austauschbar mit der Tibia-Komponente 100 lösbar verbindbar. Beispielsweise sind proximodistale Dicken von 4 mm und/oder 8 mm denkbar. Anhand der Fig. 11 und 12 ist eine lösbare Verbindung der beiden Zusatzplatte 400a', 400b' mit dem Tibia-Probeplateau 800 gezeigt. Das weitere Abstandselement 400' kann auch als Basisplatte bezeichnet werden. Die Basisplatte 400' ist wahlweise mit einer der beiden Zusatzplatten 400a', 400b' lösbar verbindbar. Zu diesem Zweck sind komplementäre Verbindungsabschnitte V, V' vorhanden. Alternativ kann die Basisplatte ohne eine der Zusatzplatten verwendet werden. Selbstverständlich kann das Instrumentensystem auch mehrere unterschiedlich dicke Basisplatten aufweisen. Die Verbindungsabschnitte V, V' sind vorzugsweise derart angeordnet und/oder gestaltet, dass die Basisplatte(n) und die Zusatzplatten ausschließlich in definierten Anordnungen miteinander verbunden und/oder übereinadergestapelt werden können.

In der anhand Fig. 11 gezeigten Konfiguration sind die erste Basisplatte 400a' und die Zusatzplatte 400' übereinandergestapelt und lösbar miteinander verbunden. Diese Konfiguration definiert eine nicht näher bezeichnete proximodistale Gesamtdicke. In der anhand Fig. 12 gezeigten Konfiguration sind die zweite Basisplatte 400b' und die Zusatzplatte 400' übereinandergestapelt und lösbar miteinander verbunden. Diese Konfiguration definiert eine nicht näher bezeichnete weitere proximodistale Gesamtdicke.

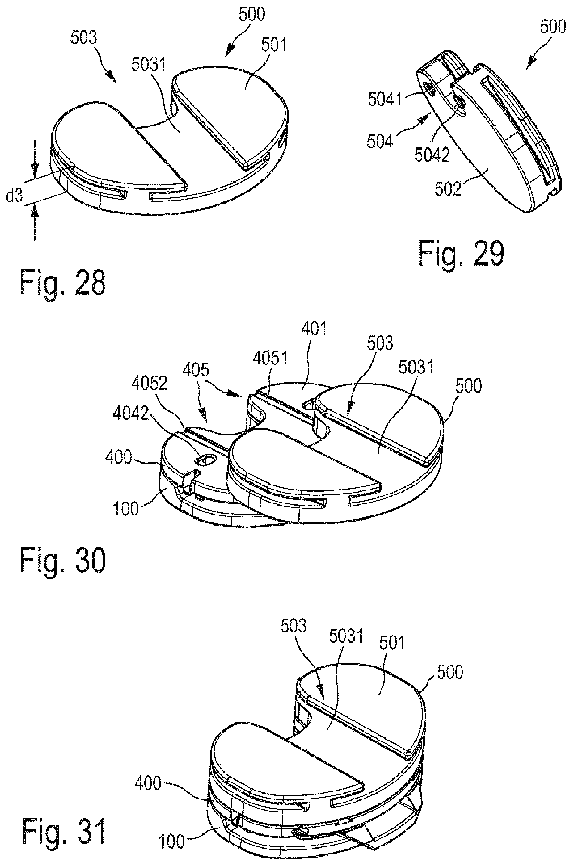

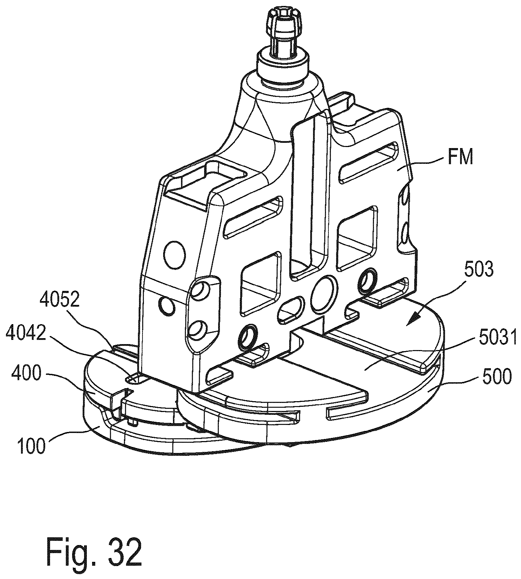

Die dritte Femur-Komponente 500 (Fig. 28, 29) weist eine Oberseite 501 und eine gegenüberliegende Unterseite 502 auf. Die Oberseite 501 ist proximal orientiert. Die Unterseite 502 ist distal orientiert. Die Oberseite 501 und die Unterseite 502 sind um eine proximodistale dritte Dicke d3 voneinander beabstandet. Die Oberseite 501 weist einen proximalen Linearführungsabschnitt 503 auf. Der proximale Linearführungsabschnitt 503 ist zur anteroposterior linearbeweglichen Führung an einem komplementären Linearführungsabschnitt eines Femur-Messblocks FM (siehe Fig. 32) eingerichtet. Die Unterseite 502 weist einen distalen Linearführungsabschnitt 504 auf. Der distale Linearführungsabschnitt 504 ist zur anteroposterior linearbeweglichen Führung an einem proximalen Linearführungsabschnitt 405 des wenigstens einen Abstandselements 400 eingerichtet (siehe Fig. 5).

Es versteht sich, dass auch die weiteren Abstandselemente 400a bis 400g jeweils über einen solchen proximalen Linearführungsabschnitt 405 verfügen. Die dritte Femur-Komponente 500 kann dementsprechend an jedem der mehreren unterschiedlichen Abstandselemente 400, 400a bis 400g linearbeweglich geführt festgelegt werden.

Bei der gezeigten Ausführungsform weist der proximale Linearführungsabschnitt 503 eine in die Oberseite 501 distal eingesenkte Führungsnut 5031 auf. Die Führungsnut 5031 ist anteroposterior längserstreckt und mediolateral hinterschnitten. Der nicht näher bezeichnete komplementäre Linearführungsabschnitt des Femur-Messblocks FM ist hierdurch proximodistal sowie mediolateral formschlüssig an/in der Führungsnut 5031 gehalten. Anteroposterior wirken die Führungsnut 5031 und der besagte komplementäre Linearführungsabschnitt des Femur-Messblocks FM gleitbeweglich zusammen.

Der distale Linearführungsabschnitt 504 weist bei der gezeigten Ausführungsform wenigstens einen von der Unterseite 502 distal aufragenden Führungsstift 5041 auf. Bei der gezeigten Ausführungsform sind zwei mediolateral voneinander beabstandete Führungsstifte 5041, 5042 vorhanden. Der hierzu komplementäre proximale Linearführungsabschnitt 405 des wenigstens einen Abstandselements 400 (und der weiteren Abstandselemente 400a bis 400g) weist dementsprechend wenigstens eine in die Oberseite 401 distal eingesenkte und anteroposterior längserstreckte Führungsnut 4051 auf. Vorliegend sind zwei mediolateral voneinander beabstandet angeordnete Führungsnuten 4051, 4052 vorhanden. Die beiden Führungsnuten 4051, 4052 sind jeweils mediolateral hinterschnitten. Die beiden Führungsstifte 5041, 5042 sind endseitig radial aufgeweitet und/oder weisen einen Pilzkopf auf. Hierdurch sind die Führungsstifte 5041, 5042 an der jeweiligen hinterschnittenen Führungsnut 4051, 4052 proximodistal und mediolateral formschlüssig gehalten. Anteroposterior wirken die Führungsstifte 5041, 5042 und die Führungsnuten 4051, 4052 jeweils gleitbeweglich zusammen.

Anhand Fig. 31 ist eine weitere unter Verwendung des modularen Instrumentensystems 1 ausgebildete Instrumentenkonfiguration gezeigt. Die Instrumentenkonfiguration nach Fig. 31 kann auch als dritte Instrumentenkonfiguration bezeichnet werden und weist die Tibia-Komponente 100, das Abstandselement 400 und die dritte Femur-Komponente 500 auf. Die dritte Femur-Komponente 500 ist auf die vorbeschriebene Weise anteroposterior linearbeweglich geführt mit dem Abstandselement 400 verbunden. Das Abstandselement 400 ist wiederum lösbar mit der Tibia-Komponente 100 verbunden.

Fig. 32 zeigt die Verwendung der Instrumentenkonfiguration nach Fig. 31 in Kombination mit dem bereits erwähnten Femur-Messblock FM. Der Femur-Messblock FM weist eine dem Fachmann bekannte Gestaltung und Funktion auf und ist in der Verwendung an dem distalen Femur des Patienten festgelegt. Die Instrumentenkonfiguration nach Fig. 31 ist wenigstens einer weiteren Operationstechnik zugeordnet und dient in erster Linie einer Ermittlung des Gelenkspalts unter Flexion.

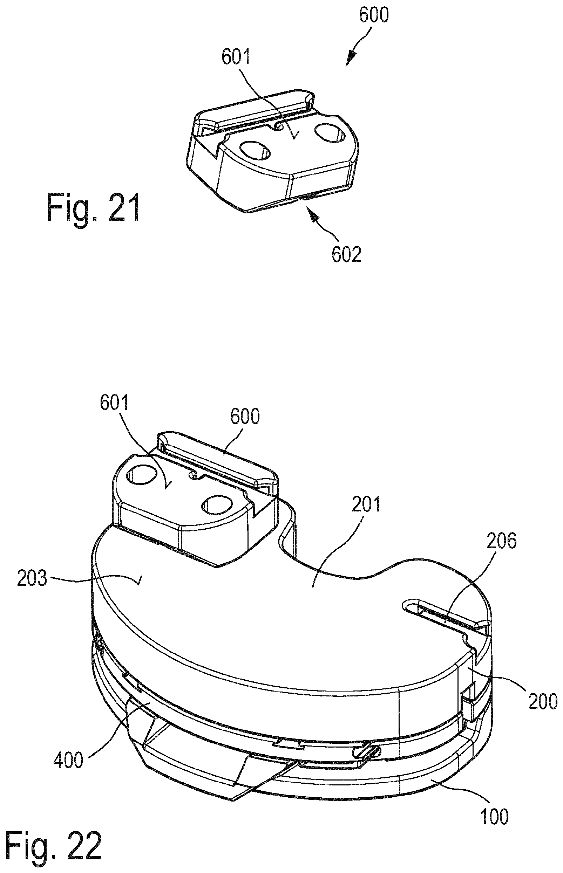

Das Augmentatselement 600 (siehe Fig. 21) weist eine proximale Augmentatsfläche 601 und einen distalen Verbindungsabschnitt 602 auf. Die proximale Augmentatsfläche 601 ist zur Anlage an dem resezierten distalen Femur und/oder resezierten posterioren Femur eingerichtet. Der distale Verbindungsabschnitt 602 ist zur lösbaren Verbindung mit einem proximalen Verbindungsabschnitt 205, 206 der ersten Femur-Komponente 200 eingerichtet. Das Augmentatselement 600 dient einem maßlichen Ausgleich etwaiger Defekte an dem Femur. Die spezifische Gestaltung des distalen Verbindungsabschnitts 602 ist im Hinblick auf die vorliegende Erfindung nicht wesentlich. Entsprechendes gilt für den distalen Verbindungsabschnitt 205, 206 der ersten Femur-Komponente. Entscheidend ist, dass die besagten Verbindungsabschnitte lösbar miteinander verbindbar sind. Dies beispielsweise unter Ausbildung einer lösbaren Steck-, Rast- und/oder Klemmverbindung.

Bei der gezeigten Ausführungsform weist die Oberseite 201 der ersten Femur-Komponente 200 mehrere proximale Verbindungsabschnitte 205, 206 zur Verbindung mit dem distalen Verbindungsabschnitt des Augmentatselements 600 auf. Die beiden proximalen Verbindungsabschnitte 205, 206 sind vorliegend mediolateral voneinander beabstandet.

Das Augmentatselement 600 kann wahlweise an einem der beiden proximalen Verbindungsabschnitte 205, 206 angebracht werden.

Fig. 22 zeigt eine weitere Instrumentenkonfiguration. Die weitere Instrumentenkonfiguration nach Fig. 22 ist unter Verwendung des modularen Instrumentensystems 1 ausgebildet und kann auch als vierte Instrumentenkonfiguration bezeichnet werden. In der vierten Instrumentenkonfiguration ist das Augmentatselement 600 an dem proximalen Verbindungsabschnitt 205 der ersten Femur-Komponente 200 lösbar festgelegt. Die zweite Femur-Komponente 200 ist wiederum lösbar mit dem Abstandselement 400 (oder alternativ einem der mehreren unterschiedlichen Abstandselemente 400a bis 400g) verbunden. Das Abstandselement 400 ist lösbar mit der Tibia-Komponente 100 verbunden.

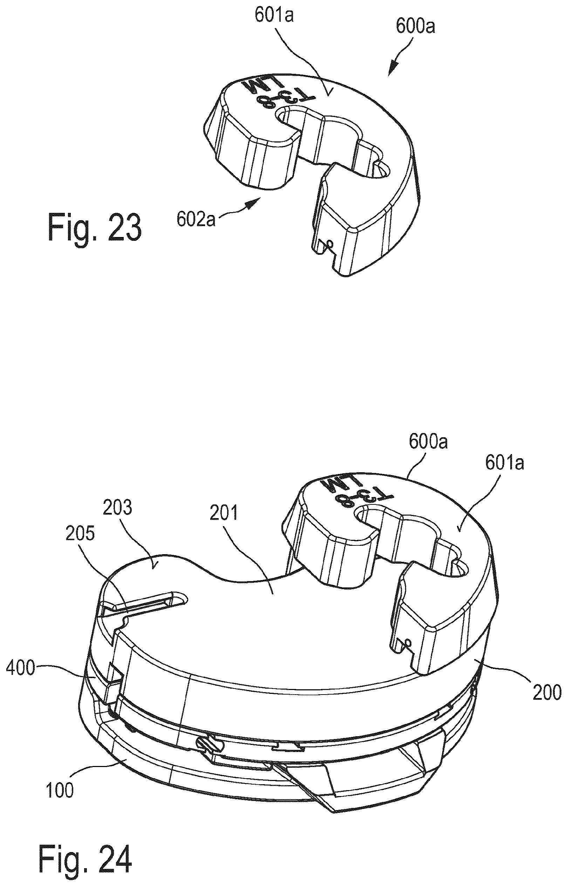

Bei der gezeigten Ausführungsform weist das modulare Instrumentensystem 1 zudem ein weiteres Augmentatselement 600a auf (siehe Fig. 23). Das weitere Augmentatselement 600a ist hinsichtlich seiner prinzipiellen Gestaltung und Funktionsweise identisch zu dem Augmentatselement 600 nach Fig. 21. Das weitere Augmentatselement 600a unterscheidet sich in erster Linie hinsichtlich seiner anteroposterioren und/oder mediolateralen und/oder proximodistalen Abmessungen von dem Augmentatselement 600. Das weitere Augmentatselement 600a kann wahlweise an einem der beiden proximalen Verbindungsabschnitte 205, 206 der ersten Femur-Komponente 200 festgelegt werden (siehe Fig. 24).

Mittels des Augmentatselements 600 und/oder des weiteren Augmentatselements 600a kann die proximodistale Gesamtdicke der jeweiligen Instrumentenkonfiguration lokal verändert werden. Lokal meint, dass die Augmentatselemente 600, 600a die Oberseite 201 der ersten Femur-Komponente 200 lediglich abschnittsweise anteroposterior und/oder mediolateral überdecken.

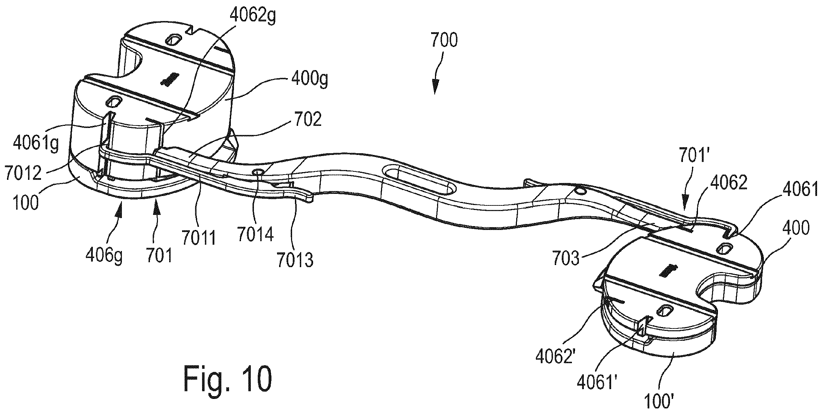

Der Handgriff 700 (siehe Fig. 1, 10) dient einer vereinfachten, ergonomischen Handhabung der jeweiligen Instrumentenkonfiguration. Mittels des Handgriffs 700 kann die Instrumentenkonfiguration ergonomisch und unter der Vermeidung von Beeinträchtigungen des Patienten in den Gelenkspalt eingebracht und aus diesem entnommen werden. Zu diesem Zweck ist der Handgriff 700 mit der jeweiligen Instrumentenkonfiguration koppelbar. Der Handgriff 700 weist hierfür vorliegend wenigstens eine manuell betätigbare Kopplungseinrichtung 701 auf. Die Kopplungseinrichtung 701 ist bei der gezeigten Ausführungsform zur lösbaren Kopplung mit einem Kopplungsabschnitt 406 des wenigstens einen Abstandselements 400 (siehe Fig. 5) eingerichtet. Es versteht sich, dass auch die weiteren Abstandselemente 400a bis 400g einen solchen Kopplungsabschnitt aufweisen.

Die wenigstens eine Kopplungseinrichtung 701 ist einends des Handgriffs 700 angeordnet und weist ein Kopplungselement 7011 auf. Das Kopplungselement 7011 ist zwischen einem ersten Ende und einem zweiten Ende (jeweils ohne Bezugszeichen) längserstreckt. An dem ersten Ende weist das Kopplungselement 7011 einen ersten Kopplungsabschnitt 7012 auf. Andernends weist das Kopplungselement 7011 einen Betätigungsabschnitt 7013 auf. Das Kopplungselement 7011 ist um eine Schwenkachse 7014 relativ zu dem Handgriff 700 schwenkbeweglich an demselben gelagert. Die Schwenkachse 7014 ist vorliegend proximodistal orientiert. Mittels einer Betätigung des Betätigungsabschnitts 7013 ist das Kopplungselement 7011 um die Schwenkachse 7014 schwenkbeweglich. Der erste Kopplungsabschnitt 7012 wird hierbei auf einer nicht näher bezeichneten Kreisbogenbahn im Wesentlichen mediolateral verlagert. Die Kopplungseinrichtung 701 weist zudem einen zweiten Kopplungsabschnitt 7021 auf. Der zweite Kopplungsabschnitt 7021 ist einends des Handgriffs 700 angeordnet (siehe Fig. 1).

Der Kopplungsabschnitt 406 des wenigstens einen Abstandselements 400 weist einen ersten Kopplungsteilabschnitt 4061 und einen zweiten Kopplungsteilabschnitt 4062 auf. Der erste Kopplungsteilabschnitt 4061 ist zum kraft- und/oder formschlüssigen Zusammenwirken mit dem ersten Koppelabschnitt 7012 der Kopplungseinrichtung 701 eingerichtet. Der erste Kopplungsteilabschnitt 4061 ist vorliegend anterior offen. Der zweite Kopplungsteilabschnitt 4062 ist zum kraft- und/oder formschlüssigen Zusammenwirken mit dem zweiten Kopplungsabschnitt 7021 der Kopplungseinrichtung 701 eingerichtet. Der zweite Kopplungsteilabschnitt 4062 ist vorliegend nach außen offen.

In Fig. 10 ist eine Situation gezeigt, in welcher die Kopplungseinrichtung 701 lösbar mit dem Koppelabschnitt 406g des (weiteren) Abstandselements 400g gekoppelt ist. Dabei greift der erste Kopplungsabschnitt 7012 medial in den ersten Kopplungsteilabschnitt 4061g ein. Der zweite Kopplungsabschnitt 7021 greift posterior in den zweiten Kopplungsteilabschnitt 4062g ein. In dem anhand Fig. 10 gezeigten Zustand ist die aus der Tibia-Komponente 100 und dem (weiteren) Abstandselement 400g gebildete Instrumentenkonfiguration mittels der Kopplungseinrichtung 701 lösbar verliersicher mit dem Handgriff 700 gekoppelt.

Bei der gezeigten Ausführungsform ist die Kopplungseinrichtung 701 an einem ersten Ende 702 des Handgriffs 700 angeordnet. Der Handgriff 700 ist zwischen dem ersten Ende 702 und einem zweiten Ende 703 längserstreckt. Das zweite Ende 703 weist vorliegend eine weitere Kopplungseinrichtung 701' auf. Die Funktion und der Aufbau der weiteren Kopplungseinrichtung 701' sind identisch mit der Kopplungseinrichtung 701. In der anhand Fig. 10 gezeigten Situation ist eine aus einer weiteren Tibia-Komponente 100' und dem Abstandselement 400 gebildete Instrumentenkonfiguration mittels der weiteren Kopplungseinrichtung 701' lösbar mit dem Handgriff 700 gekoppelt.

Wie weiter anhand Fig. 5 gezeigt ist, weist das Abstandselement 400 einen weiteren komplementären Kopplungsabschnitt 406' auf. Der weitere Kopplungsabschnitt 406' ist in Bezug auf eine sagittale Mittellängsebene des Abstandselements 400 spiegelsymmetrisch zu dem Kopplungsabschnitt 406 gestaltet. Hierdurch kann die Kopplungseinrichtung 701 und/oder die weitere Kopplungseinrichtung 701' des Handgriffs 700 wahlweise an einem der beiden Kopplungsabschnitte 406, 406' festgelegt werden.

Der Handgriff 700 ist bei der gezeigten Ausführungsform zwischen dem ersten Ende 702 und dem zweite Ende 703, insbesondere S-förmig, gebogen längserstreckt. Vorliegend ist das Instrumentensystem 1 sowohl an einem rechten Knie als auch einem linken Knie verwendbar. Die Biegung des Handgriffs 700, das Vorhandensein der zwei, insbesondere symmetrisch angeordneten, Kopplungseinrichtungen 701, 701' und der symmetrisch angeordneten Kopplungsabschnitte 406, 406' dient insbesondere der erwähnten beidseitigen Verwendbarkeit.

Das Tibia-Probeplateau 800 (siehe Fig. 3) weist eine Oberseite 801 und eine Unterseite 802 auf. Die Oberseite 801 ist proximal orientiert. Die Unterseite 802 ist distal orientiert. Die Unterseite 802 weist eine Befestigungsfläche 803 auf. Die Befestigungsfläche 803 ist zur Befestigung an der resezierten proximalen Tibia eingerichtet. Zu diesem Zweck weist das Tibia-Probeplateau 800 mehrere nicht näher bezeichnete und jeweils zwischen der Oberseite 801 und der Unterseite 802 durchgängig erstreckte Aufnahmebohrungen für Befestigungspins P auf. Mittels der Befestigungspins P kann das Tibia-Probeplateau 800 auf eine dem Fachmann bekannte Weise tibiaseitig befestigt werden. Die Oberseite 801 weist einen proximalen Verbindungsabschnitt 804 auf. Der proximale Verbindungsabschnitt 804 ist bei der gezeigten Ausführungsform hinsichtlich seiner Funktion und Gestaltung im Wesentlichen identisch mit dem proximalen Verbindungsabschnitt 104 der Tibia-Komponente 100 (siehe Fig. 2). Dementsprechend ist der proximale Verbindungsabschnitt 804 zur lösbaren Verbindung mit dem distalen Verbindungsabschnitt 403 des wenigstens einen Abstandselements 400 (siehe Fig. 6) oder jedes der weiteren Abstandselemente 400a bis 400g eingerichtet. Der proximale Verbindungsabschnitt 804 weist wiederum zwei mediolateral voneinander beabstandete Verbindungsteilabschnitte 8041, 8042 auf. Zur Vermeidung von Wiederholungen wird auf das zu den Verbindungsteilabschnitten 1041, 1042 Gesagte verwiesen und ausdrücklich Bezug genommen.

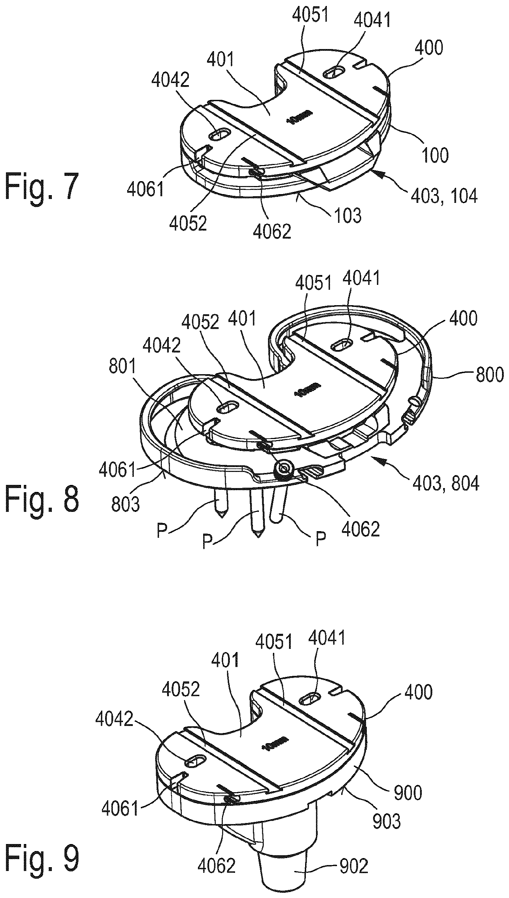

Bei der gezeigten Ausführungsform des modularen Instrumentensystems 1 sind mehrere unterschiedlich große Tibia-Probeplateaus 800, 800a, 800b vorhanden. Die weiteren Tibia-Probeplateaus 800a, 800b sind nicht gesondert dargestellt und hinsichtlich ihres grundsätzlichen Aufbaus und ihrer Funktion identisch zu dem Tibia-Probeplateau 800. Lediglich deren anteroposteriore und/oder mediolaterale Abmessungen sind unterschiedlich. Die mehreren Tibia-Probeplateaus 800, 800a, 800b sind gegeneinander austauschbar. So beispielsweise in Bezug auf die anhand Fig. 8 gezeigte Instrumentenkonfiguration, in welcher das wenigstens eine Abstandselement 400 lösbar mit dem Tibia-Probeplateau 800 verbunden ist. Anstelle des Tibia-Probeplateaus 800 kann eines der besagten weiteren Tibia-Probeplateaus 800a, 800b verwendet werden. Bei einer Ausgestaltung sind insgesamt 9 unterschiedlich große Tibia-Probeplateaus vorhanden.

Das Tibia-Plateau 900 (siehe Fig. 4) weist eine Oberseite 901 und einen distalen Verankerungszapfen 902 auf. Der distale Verankerungszapfen 902 ragt distal von einer Unterseite 903 auf. Die Oberseite 901 weist eine proximalen Verbindungsabschnitt 904 auf. Der proximale Verbindungsabschnitt 904 ist zur lösbaren Verbindung mit der Unterseite 402 des wenigstens einen Abstandselements 400 (oder jedes der weiteren Abstandselemente 400a bis 400g) eingerichtet.

Anhand Fig. 9 ist eine weitere Instrumentenkonfiguration gezeigt, in welcher das Abstandselement 400 lösbar mit dem Tibia-Plateau 900 verbunden ist.

Bei der gezeigten Ausführungsform des modularen Instrumentensystems 1 sind mehrere unterschiedlich große Tibia-Plateaus 900, 900a, 900b vorhanden. Die Tibia-Plateaus 900, 900a, 900b unterscheiden sich lediglich hinsichtlich ihrer anteroposterioren und/oder mediolateralen Abmessungen und sind zur Implantation in unterschiedlich große Tibiaknochen vorgesehen. Die weiteren Tibia-Plateaus 900a, 900b sind in den Figuren nicht im Detail gezeigt. Die unterschiedlichen Tibia-Plateaus 900, 900a, 900b sind gegeneinander austauschbar. So beispielsweise in Bezug auf die anhand Fig. 9 gezeigte Instrumentenkonfiguration. Bei einer Ausgestaltung sind insgesamt 9 unterschiedlich große Tibia-Plateaus vorhanden.

## Patentansprüche

1. Modulares Instrumentensystem (1) zur Verwendung bei einer Kniegelenkersatzoperation, aufweisend
wenigstens eine Tibia-Komponente (100) mit einer Oberseite (101) und einer gegenüberliegenden Unterseite (102), wobei die Unterseite (102) eine distal orientierte ebene Anlagefläche (103) aufweist, welche zur Anlage an einer resezierten proximalen Tibia eingerichtet ist,
eine erste Femur-Komponente (200) mit einer Oberseite (201) und einer parallel gegenüberliegenden Unterseite (202), wobei die Oberseite (201) eine proximal orientierte ebene erste Anlagefläche (203) aufweist, welche um eine proximodistale erste Dicke (d1) von der Unterseite (202) beabstandet und zur Anlage an einem resezierten Femur eingerichtet ist,
eine zweite Femur-Komponente (300) mit einer Oberseite (301) und einer parallel gegenüberliegenden Unterseite (302), wobei die Oberseite (301) eine proximal orientierte ebene zweite Anlagefläche (303) aufweist, welche um eine proximodistale zweite Dicke (d2) von der Unterseite (302) beabstandet und zur Anlage an einem distalen Femur-Sägeblock und zur alternativen Anlage an einem posterioren Femur-Sägeblock eingerichtet ist,
wenigstens ein Abstandselement (400) mit einer Oberseite (401) und einer um eine proximodistale Abstandsdicke (t) parallel beabstandete Unterseite (402), wobei die Unterseite (402) des Abstandselements (400) einen distalen Verbindungsabschnitt (403) aufweist, welcher zur lösbaren Verbindung mit einem komplementären proximalen Verbindungsabschnitt (104) der Oberseite (101) der wenigstens einen Tibia-Komponente (100) eingerichtet ist, wobei die Oberseite (401) des Abstandselements (400) einen proximalen Verbindungsabschnitt (404) aufweist, welcher zur lösbaren Verbindung mit einem komplementären distalen Verbindungsabschnitt (204) der Unterseite (202) der ersten Femur-Komponente (200) und zur alternativen lösbaren Verbindung mit einem komplementären distalen Verbindungsabschnitt (304) der Unterseite (302) der zweiten Femur-Komponente (300) eingerichtet ist,
wobei das modulare Instrumentensystem (1) zum Ausbilden wenigstens einer ersten Instrumentenkonfiguration und einer zweiten Instrumentenkonfiguration eingerichtet ist, wobei in der ersten Instrumentenkonfiguration die erste Femur-Komponente (200) und das Abstandselement (400) miteinander lösbar verbunden sind, und wobei in der zweiten Instrumentenkonfiguration die zweite Femur-Komponente (300) und das Abstandselement (400) miteinander lösbar verbunden sind.

2. Modulares Instrumentensystem (1) nach Anspruch 1, **dadurch gekennzeichnet, dass** die erste Dicke (d1) größer ist als die zweite Dicke (d2).

3. Modulares Instrumentensystem (1) nach Anspruch 1 oder 2, **dadurch gekennzeichnet, dass** mehrere unterschiedlich dicke Abstandselemente (400, 400a bis 400g, 400', 400a', 400b') mit unterschiedlichen Abstandsdicken (t, ta bis tg) vorhanden sind, wobei die mehreren unterschiedlich dicken Abstandselemente (400, 400a bis 400g, 400', 400a', 400b') gegeneinander austauschbar einzeln verwendbar und/oder miteinander kombiniert übereinandergestapelt verwendbar sind.

4. Modulares Instrumentensystem (1) nach einem der vorhergehenden Ansprüche, **dadurch gekennzeichnet, dass** eine dritte Femur-Komponente (500) vorhanden ist und eine Oberseite (501) und eine um eine proximodistale dritte Dicke (d3) parallel beabstandete Unterseite (502) aufweist, wobei die Oberseite einen proximalen Linearführungsabschnitt (503) aufweist, welcher zur anteroposterior linearbeweglichen Führung an einem komplementären Linearführungsabschnitt eines Femur-Messblocks (FM) eingerichtet ist, wobei die Unterseite (502) einen distalen Linearführungsabschnitt (504) aufweist, welcher zur anteroposterior linearbeweglichen Führung an einem proximalen Linearführungsabschnitt (405) der Oberseite (401) des wenigstens einen Abstandselements (400) eingerichtet ist, und wobei das modulare Instrumentensystem (1) zum Ausbilden einer dritten Instrumentenkonfiguration eingerichtet ist, in welcher die dritte Femur-Komponente (500) und das wenigstens eine Abstandselement (400) mittels ihrer komplementären Linearführungsabschnitte (504, 405) lösbar relativbeweglich miteinander verbunden sind.

5. Modulares Instrumentensystem (1) nach Anspruch 4, **dadurch gekennzeichnet, dass** der proximale Linearführungsabschnitt (503) der dritten Femur-Komponente (500) wenigstens eine in die Oberseite (501) distal eingesenkte und anteroposterior längserstreckte sowie mediolateral hinterschnittene Führungsnut (5031) aufweist.

6. Modulares Instrumentensystem (1) nach Anspruch 4 oder 5, **dadurch gekennzeichnet, dass** der distale Linearführungsabschnitt (504) der dritten Femur-Komponente (500) wenigstens einen von der Unterseite (502) distal aufragenden Führungsstift (5041, 5042) aufweist, und dass der proximale Linearführungsabschnitt (405) des wenigstens einen Abstandselements (400) wenigstens eine in die Oberseite (401) distal eingesenkte und anteroposterior längserstreckte sowie mediolateral hinterschnittene Führungsnut (4051, 4052) aufweist.

7. Modulares Instrumentensystem (1) nach einem der vorhergehenden Ansprüche, **dadurch gekennzeichnet, dass** wenigstens ein Augmentatselement (600) vorhanden ist und eine proximale Augmentatsfläche (601) zur Anlage an dem resezierten Femur und einen distalen Verbindungsabschnitt (602) aufweist, welcher zur lösbaren Verbindung mit einem proximalen Verbindungsabschnitt (205, 206) der ersten Femur-Komponente (200) eingerichtet ist, wobei das modulare Instrumentensystem (1) zum Ausbilden einer vierten Instrumentenkonfiguration eingerichtet ist, in welcher das Augmentatselement (600) an der ersten Anlagefläche (203) der ersten Femur-Komponente (200) angebracht ist.

8. Modulares Instrumentensystem (1) nach Anspruch 7, **dadurch gekennzeichnet, dass** die erste Femur-Komponente (200) mehrere proximale Verbindungsabschnitte (205, 206) aufweist, die jeweils in die Oberseite (201) der ersten Femur-Komponente (200) eingesenkt und mediolateral und/oder anteroposterior voneinander beabstandet positioniert sind.

9. Modulares Instrumentensystem (1) nach Anspruch 7 oder 8, **dadurch gekennzeichnet, dass** mehrere unterschiedlich geformte und/oder unterschiedlich große Augmentatselemente (600, 600a) vorhanden und gegeneinander austauschbar verwendbar sind.

10. Modulares Instrumentensystem (1) nach einem der vorhergehenden Ansprüche, **dadurch gekennzeichnet, dass** ein Handgriff (700) vorhanden ist und wenigstens eine manuell betätigbare Kopplungseinrichtung (701) aufweist, welche zur lösbaren Kopplung mit einem Kopplungsabschnitt (406) des wenigstens einen Abstandselements (400) eingerichtet ist.

11. Modulares Instrumentensystem (1) nach Anspruch 10, **dadurch gekennzeichnet, dass** der Handgriff (700) zwischen einem ersten Ende (702) und einem zweiten Ende (703) längserstreckt ist und an beiden Enden (702, 703) jeweils eine Kopplungseinrichtung (701, 701') aufweist.

12. Modulares Instrumentensystem (1) nach einem der vorhergehenden Ansprüche, **dadurch gekennzeichnet, dass** wenigstens ein Tibia-Probeplateau (800) vorhanden ist und eine Oberseite (801) und eine distal gegenüberliegende Unterseite (802) aufweist, wobei die Unterseite (802) eine distal orientierte Befestigungsfläche (803) zur Befestigung an der resezierten proximalen Tibia aufweist, wobei die Oberseite (801) einen proximalen Verbindungsabschnitt (804) aufweist, welcher zur lösbaren Verbindung mit dem distalen Verbindungsabschnitt (403) des wenigstens einen Abstandselements (400) eingerichtet ist, und wobei das modulare Instrumentensystem (1) zum Ausbilden einer fünften Instrumentenkonfiguration eingerichtet ist, in welcher das wenigstens eine Abstandselement (400) und das Tibia-Probeplateau (800) miteinander lösbar verbunden sind.

13. Modulares Instrumentensystem (1) nach Anspruch 12, **dadurch gekennzeichnet, dass** mehrere unterschiedlich große Tibia-Probeplateaus (800, 800a, 800b) vorhanden und gegeneinander austauschbar verwendbar sind.

14. Modulares Instrumentensystem (1) nach einem der vorhergehenden Ansprüche, **dadurch gekennzeichnet, dass** wenigstens ein Tibia-Plateau (900) vorhanden ist und eine Oberseite (901) und einen distalen Verankerungszapfen (902) zur Verankerung an der resezierten proximalen Tibia aufweist, wobei die Oberseite (901) einen proximalen Verbindungsabschnitt (904) aufweist, welcher zur lösbaren Verbindung mit dem distalen Verbindungsabschnitt (403) des wenigstens einen Abstandselements (400) eingerichtet ist, und wobei das modulare Instrumentensystem (1) zum Ausbilden einer sechsten Instrumentenkonfiguration eingerichtet ist, in welcher das wenigstens eine Abstandselement (400) und das Tibia-Plateau (900) miteinander lösbar verbunden sind.

15. Modulares Instrumentensystem (1) nach Anspruch 14, **dadurch gekennzeichnet, dass** mehrere unterschiedlich große Tibia-Plateaus (900, 900a, 900b) vorhanden und gegeneinander austauschbar verwendbar sind.

## Claims

1. Modular instrument system (1) for use in a knee joint replacement operation, comprising
at least one tibial component (100) having a topside (101) and an opposite underside (102), wherein the underside (102) has a distally oriented flat contact surface (103), which is configured to bear on a resected proximal tibia,
a first femoral component (200) having a topside (201) and a parallel opposite underside (202), wherein the topside (201) has a proximally oriented, flat first contact surface (203), which is spaced apart from the underside (202) by a proximodistal first thickness (d1) and is configured to bear on a resected femur,
a second femoral component (300) having a topside (301) and a parallel opposite underside (302), wherein the topside (301) has a proximally oriented, flat second contact surface (303), which is spaced apart from the underside (302) by a proximodistal second thickness (d2) and is configured to bear on a distal femoral cutting jig and to alternatively bear on a posterior femoral cutting jig,
at least one spacer element (400) having a topside (401) and an underside (402) spaced apart in parallel by a proximodistal spacing thickness (t), wherein the underside (402) of the spacer element (400) has a distal connecting portion (403), which is configured for releasable connection to a complementary proximal connecting portion (104) of the topside (101) of the at least one tibial component (100), wherein the topside (401) of the spacer element (400) has a proximal connecting portion (404), which is configured for releasable connection to a complementary distal connecting portion (204) of the underside (202) of the first femoral component (200) and for alternative releasable connection to a complementary distal connecting portion (304) of the underside (302) of the second femoral component (300),
wherein the modular instrument system (1) is configured for forming at least a first instrument configuration and a second instrument configuration, wherein in the first instrument configuration the first femoral component (200) and the spacer element (400) are releasably connected to each other, and wherein in the second instrument configuration the second femoral component (300) and the spacer element (400) are releasably connected to each other.

2. Modular instrument system (1) according to Claim 1, **characterized in that** the first thickness (d1) is greater than the second thickness (d2).

3. Modular instrument system (1) according to Claim 1 or 2, **characterized in that** a plurality of spacer elements of different thicknesses (400, 400a to 400g, 400', 400a',400b') with different spacing thicknesses (t, ta to tg) are present, wherein the plurality of spacer elements of different thicknesses (400, 400a to 400g, 400', 400a', 400b') can be interchangeably used individually and/or can be used in combination stacked on top of one another.

4. Modular instrument system (1) according to one of the preceding claims, **characterized in that** a third femoral component (500) is present and has a topside (501) and an underside (502) spaced apart in parallel by a proximodistal third thickness (d3), wherein the topside (501) has a proximal linear guide portion (503), which is configured for anteroposterior linearly movable guiding on a complementary linear guide portion of a femoral measuring block (FM), wherein the underside (502) has a distal linear guide portion (504), which is configured for anteroposterior linearly movable guiding on a proximal linear guide portion (405) of the topside (401) of the at least one spacer element (400), and wherein the modular instrument system (1) is configured for forming a third instrument configuration, in which the third femoral component (500) and the at least one spacer element (400) are connected releasably and movably relative to each other by means of their complementary linear guide portions (504, 405).

5. Modular instrument system (1) according to Claim 4, **characterized in that** the proximal linear guide portion (503) of the third femoral component (500) has at least one guide groove (5031) which is distally recessed into the topside (501), extends in the anteroposterior direction and is undercut mediolaterally.

6. Modular instrument system (1) according to Claim 4 or 5, **characterized in that** the distal linear guide portion (504) of the third femoral component (500) has at least one guide pin (5041, 5042) protruding distally from the underside (502), and **in that** the proximal linear guide portion (405) of the at least one spacer element (400) has at least one guide groove (4051, 4052) which is distally recessed into the topside (401), extends in the anteroposterior direction and is undercut mediolaterally.

7. Modular instrument system (1) according to one of the preceding claims, **characterized in that** at least one augmentation element (600) is present and has a proximal augmentation surface (601) for bearing on the resected femur and a distal connecting portion (602) which is configured for releasable connection to a proximal connecting portion (205, 206) of the first femoral component (200), wherein the modular instrument system (1) is configured for forming a fourth instrument configuration, in which the augmentation element (600) is mounted on the first contact surface (203) of the first femoral component (200).

8. Modular instrument system (1) according to Claim 7, **characterized in that** the first femoral component (200) has a plurality of proximal connecting portions (205, 206), which are each recessed into the topside (201) of the first femoral component (200) and spaced apart mediolaterally and/or anteroposteriorly from each other.

9. Modular instrument system (1) according to Claim 7 or 8, **characterized in that** a plurality of differently shaped and/or different-sized augmentation elements (600, 600a) are present and can be used interchangeably with each other.

10. Modular instrument system (1) according to one of the preceding claims, **characterized in that** a handle (700) is present and has at least one manually actuated coupling device (701), which is configured for releasable coupling to a coupling portion (406) of the at least one spacer element (400).

11. Modular instrument system (1) according to Claim 10, **characterized in that** the handle (700) extends between a first end (702) and a second end (703) and has a respective coupling device (701, 701') at both ends (702, 703).

12. Modular instrument system (1) according to one of the preceding claims, **characterized in that** at least one tibial trial plateau (800) is present and has a topside (801) and a distally opposite underside (802), wherein the underside (802) has a distally oriented fastening surface (803) for fastening to the resected proximal tibia, wherein the topside (801) has a proximal connecting portion (804) which is configured for releasable connection to the distal connecting portion (403) of the at least one spacer element (400), and wherein the modular instrument system (1) is configured for forming a fifth instrument configuration, in which the at least one spacer element (400) and the tibial trial plateau (800) are releasably connected to each other.

13. Modular instrument system (1) according to Claim 12, **characterized in that** a plurality of different-sized tibial trial plateaus (800, 800a, 800b) are present and can be used interchangeably with one another.

14. Modular instrument system (1) according to one of the preceding claims, **characterized in that** at least one tibial plateau (900) is present and has a topside (901) and a distal anchoring pin (902) for anchoring to the resected proximal tibia, wherein the topside (901) has a proximal connecting portion (904) which is configured for releasable connection to the distal connecting portion (403) of the at least one spacer element (400), and wherein the modular instrument system (1) is configured for forming a sixth instrument configuration, in which the at least one spacer element (400) and the tibial plateau (900) are releasably connected to each other.

15. Modular instrument system (1) according to Claim 14, **characterized in that** a plurality of different-sized tibial plateaus (900, 900a, 900b) are present and can be used interchangeably with one another.

## Revendications

1. Système d'instrument modulaire (1) destiné à être utilisé lors d'une opération de remplacement de l'articulation du genou, comportant
au moins un composant tibial (100) avec une face supérieure (101) et une face inférieure (102) opposée, la face inférieure (102) comportant une surface d'appui plane orientée distalement (103), qui est mise au point pour venir en appui sur un tibia proximal réséqué,
un premier composant fémoral (200) avec une face supérieure (201) et une face inférieure (202) parallèlement opposée, la face supérieure (201) comportant une première surface d'appui plane orientée de manière proximale (203), qui est espacée de la face inférieure (202) d'une première épaisseur proximodistale (d1) et est mise au point pour venir en appui sur un fémur réséqué,
un deuxième composant fémoral (300) avec une face supérieure (301) et une face inférieure (302) parallèlement opposée, la face supérieure (301) comportant une deuxième surface d'appui plane orientée de manière proximale (303), qui est espacée de la face inférieure (302) d'une deuxième épaisseur proximodistale (d2) et est mise au point pour venir en appui sur un bloc de scie fémorale distal et pour venir en appui en variante sur un bloc de scie fémorale postérieur,
au moins un élément d'écartement (400) avec une face supérieure (401) et une face inférieure (402) espacée parallèlement d'une épaisseur d'écartement proximodistale (t), la face inférieure (402) de l'élément d'écartement (400) comportant une section de liaison distale (403), qui est mise au point pour la liaison amovible à une section de liaison proximale complémentaire (104) de la face supérieure (101) de l'au moins un composant tibial (100), la face supérieure (401) de l'élément d'écartement (400) comportant une section de liaison proximale (404), qui est mise au point pour la liaison amovible à une section de liaison distale complémentaire (204) de la face inférieure (202) du premier composant fémoral (200) et pour la liaison amovible en variante à une section de liaison distale complémentaire (304) de la face inférieure (302) du deuxième composant fémoral (300),
le système d'instrument modulaire (1) étant mis au point pour former au moins une première configuration d'instrument et une deuxième configuration d'instrument, le premier composant fémoral (200) et l'élément d'écartement (400) étant reliés l'un à l'autre de manière amovible dans la première configuration d'instrument, et le deuxième composant fémoral (300) et l'élément d'écartement (400) étant reliés l'un à l'autre de manière amovible dans la deuxième configuration d'instrument.

2. Système d'instrument modulaire (1) selon la revendication 1, **caractérisé en ce que** la première épaisseur (d1) est supérieure à la deuxième épaisseur (d2).

3. Système d'instrument modulaire (1) selon la revendication 1 ou 2, **caractérisé en ce que** plusieurs éléments d'écartement (400, 400a à 400g, 400', 400a', 400b') d'épaisseurs d'écartement différentes (t, ta à tg) sont présents, les plusieurs éléments d'écartement (400, 400a à 400g, 400', 400a', 400b') d'épaisseur différente pouvant être utilisés individuellement de manière interchangeable et/ou pouvant être utilisés de manière empilée les uns au-dessus des autres dans une combinaison entre eux.

4. Système d'instrument modulaire (1) selon l'une des revendications précédentes, **caractérisé en ce qu'**un troisième composant fémoral (500) est présent et comporte une face supérieure (501) et une face inférieure (502) espacée parallèlement d'une troisième épaisseur proximale (d3), la face supérieure comportant une section de guidage linéaire proximale (503), qui est mise au point pour le guidage antéropostérieur à déplacement linéaire sur une section de guidage linéaire complémentaire d'un bloc de mesure fémoral (FM), la face inférieure (502) comportant une section de guidage linéaire distale (504), qui est mise au point pour le guidage antéropostérieur à déplacement linéaire sur une section de guidage linéaire proximale (405) de la face supérieure (401) de l'au moins un élément d'écartement (400), et le système d'instrument modulaire (1) étant mis au point pour former une troisième configuration d'instrument, dans laquelle le troisième composant fémoral (500) et l'au moins un élément d'écartement (400) sont reliés l'un à l'autre de manière amovible et relativement mobile au moyen de leurs sections de guidage linéaire complémentaires (504, 405).

5. Système d'instrument modulaire (1) selon la revendication 4, **caractérisé en ce que** la section de guidage linéaire proximale (503) du troisième composant fémoral (500) comporte au moins une rainure de guidage (5031) enfoncée distalement dans la face supérieure (501) et s'étendant longitudinalement antéropostérieurement et contre-dépouillée médiolatéralement.

6. Système d'instrument modulaire (1) selon la revendication 4 ou 5, **caractérisé en ce que** la section de guidage linéaire distale (504) du troisième composant fémoral (500) comporte au moins une broche de guidage (5041, 5042) dépassant distalement de la face inférieure (502), et que la section de guidage linéaire proximale (405) de l'au moins un élément d'écartement (400) comporte au moins une rainure de guidage (4051, 4052) enfoncée distalement dans la face supérieure (401) et s'étendant longitudinalement antéropostérieurement et contre-dépouille médiolatéralement.

7. Système d'instrument modulaire (1) selon l'une des revendications précédentes, **caractérisé en ce qu'**au moins un élément d'augmentation (600) est présent et comporte une surface d'augmentation proximale (601) destinée à venir en appui sur le fémur réséqué et une section de liaison distale (602), qui est mise au point pour une liaison amovible à une section de liaison proximale (205, 206) du premier composant fémoral (200), le système d'instrument modulaire (1) étant mis au point pour former une quatrième configuration d'instrument, dans laquelle l'élément d'augmentation (600) est fixé sur la première surface d'appui (203) du premier composant fémoral (200).

8. Système d'instrument modulaire (1) selon la revendication 7, **caractérisé en ce que** le premier composant fémoral (200) comporte plusieurs sections de liaison proximales (205, 206), qui sont chacune enfoncées dans la face supérieure (201) du premier composant fémoral (200) et sont positionnées à distance les unes des autres sur le plan médiolatéral et/ou antéropostérieur.

9. Système d'instrument modulaire (1) selon la revendication 7 ou 8, **caractérisé en ce que** plusieurs éléments d'augmentation (600, 600a) de formes et/ou de dimensions différentes sont présents et peuvent être utilisés de manière interchangeable.

10. Système d'instrument modulaire (1) selon l'une des revendications précédentes, **caractérisé en ce qu'**une poignée (700) est prévue et comporte au moins un dispositif d'accouplement (701) pouvant être actionné manuellement, qui est mis au point pour l'accouplement amovible à une section d'accouplement (406) de l'au moins un élément d'écartement (400).

11. Système d'instrument modulaire (1) selon la revendication 10, **caractérisé en ce que** la poignée (700) s'étend longitudinalement entre une première extrémité (702) et une seconde extrémité (703) et comporte un dispositif d'accouplement (701, 701') sur chacune des deux extrémités (702, 703).

12. Système d'instrument modulaire (1) selon l'une des revendications précédentes, **caractérisé en ce qu'**au moins un plateau d'échantillon de tibia (800) est présent et comporte une face supérieure (801) et une face inférieure opposée distalement (802), la face inférieure (802) comportant une surface de fixation (803) orientée distalement pour la fixation sur le tibia proximal réséqué, la face supérieure (801) comportant une section de liaison proximale (804), qui est mise au point pour une liaison amovible à la section de liaison distale (403) de l'au moins un élément d'écartement (400), et le système d'instrument modulaire (1) étant mis au point pour former une cinquième configuration d'instrument, dans laquelle l'au moins un élément d'écartement (400) et le plateau d'échantillon de tibia (800) sont reliés de manière amovible l'un à l'autre.

13. Système d'instrument modulaire (1) selon la revendication 12, **caractérisé en ce que** plusieurs plateaux d'échantillon de tibia (800, 800a, 800b) de tailles différentes sont présents et peuvent être utilisés de manière interchangeable.

14. Système d'instrument modulaire (1) selon l'une des revendications précédentes, **caractérisé en ce qu'**au moins un plateau de tibia (900) est présent et comporte une face supérieure (901) et un tenon d'ancrage distal (902) pour l'ancrage sur le tibia proximal réséqué, la face supérieure (901) comportant une section de liaison proximale (904), qui est mise au point pour la liaison amovible à la section de liaison distale (403) de l'au moins un élément d'écartement (400), et le système d'instrument modulaire (1) étant mis au point pour former une sixième configuration d'instrument, dans laquelle l'au moins un élément d'écartement (400) et le plateau de tibia (900) sont reliés de manière amovible l'un à l'autre.

15. Système d'instrument modulaire (1) selon la revendication 14, **caractérisé en ce que** plusieurs plateaux de tibia (900, 900a, 900b) de tailles différentes sont présents et peuvent être utilisés de manière interchangeable.
